# EUROPÄISCHE PATENTSCHRIFT

(11) **EP 0 453 405 B1**
(45) Veröffentlichungstag und Bekanntmachung des Hinweises auf die Patenterteilung: **12.07.2000**
(21) Anmeldenummer: 91810269.0
(22) Anmeldetag: 11.04.1991
(51) Int. Cl.: C07D 401/12, C07D 401/14, C08K 5/3435, C08K 5/3492

(54) **Verbindungen aus o-Hydroxyphenyl-1,3,5-triazinen und sterisch gehinderten Aminen**
Compounds containing o-hydroxyphenyl-1,3,5-triazines and sterile inhibited amines
Composés à fonction amine stériquement encombrée et à fonction o-hyroxyphényl-1,3,5-triazines

(30) Priorität: 20.04.1990 CH 133890
(43) Veröffentlichungstag der Anmeldung: 23.10.1991
(73) Patentinhaber: Ciba Specialty Chemicals Holding Inc., 4057 Basel (CH)
(72) Erfinder: Slongo, Mario, Dr., CH-1712 Tafers (CH); Birbaum, Jean-Luc, Dr., CH-1700 Fribourg (CH); Rody, Jean, Dr., CH-4125 Riehen (CH); Valet, Andreas, Dr., W-7859 Eimeldingen (DE)

(56) Entgegenhaltungen:
- EP-A- 0 010 516
- EP-A- 0 200 190
- EP-A- 0 389 427
- US-A- 4 745 194

## Beschreibung

Die vorliegende Erfindung betrifft neue Verbindungen aus o-Hydroxyphenyl-1,3,5-triazinen und sterisch gehinderten Aminen, deren Verwendung zum Stabilisieren von organischen Materialien sowie die mit den neuen Verbindungen stabilisierten organischen Materialien.

Die US-A 4 619 956 beschreibt die Verwendung von synergistischen Gemischen aus einer 2,2,6,6-Tetraalkylpiperidinverbindung, deren Säuresalzen oder Komplexen mit Metallen und bestimmten o-Hydroxyphenyl-s-triazinen zum Stabilisieren von Polymerfilmen, -beschichtungen und -formkörpern.

Aus der US-A 4 289 686 sind Verbindungen, die in ihrem Molekül mindestens eine 2-(2'-Hydroxyphenyl)-benztriazol- oder 2-Hydroxybenzophenon-Gruppe und mindestens eine Polyalkylpiperidingruppe enthalten, als Lichtschutzmittel bekannt.

Gegenstand der Erfindung sind neue Verbindungen der Formel Ia 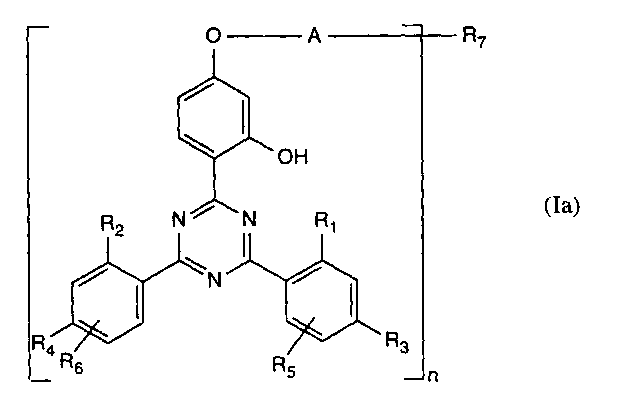 worin n 1 ist,
R₁ und R₂ unabhängig voneinander Wasserstoff, -OH, C₁-C₁₂-Alkyl oder Halogen,
R₃ und R₄ unabhängig voneinander Wasserstoff, -OH, C₁-C₁₂-Alkyl, C₁-C₁₈-Alkoxy, Halogen oder einen Rest -O-A-R₇ darstellen,
R₅ und R₆ unabhängig voneinander Wasserstoff, C₁-C₁₂-Alkyl, Halogen oder einen Rest -O-A-R₇ bedeuten,
A eine direkte Bindung, -(CH₂)ₘCO- mit m = Null oder 1 bis 4, -CH₂CH(R')O- oder -CH₂CH(OH)CH₂- ist,
R₇ ein Rest der Formeln V bis VIIb oder Xa oder Xb ist: 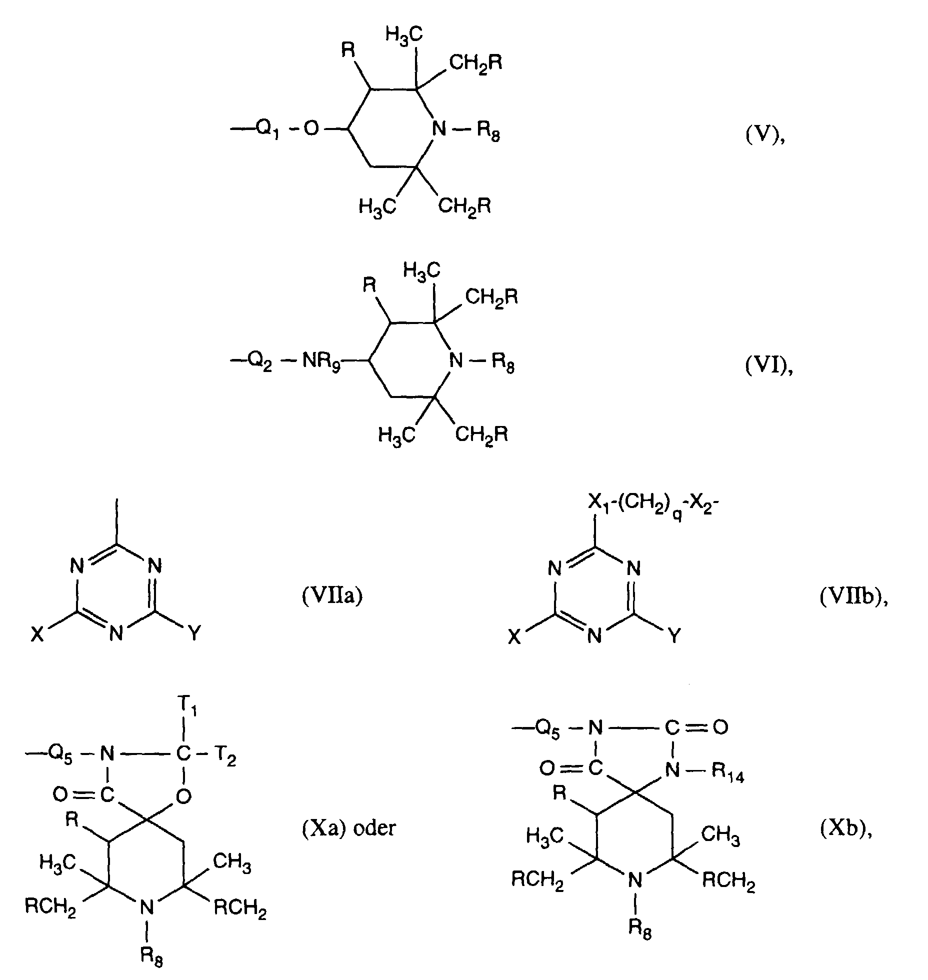 wobei Reste der Formeln VIIa nur in Verbindungen der Formel Ia mit A = direkte Bindung oder -CH₂CH(R')O- und Reste der Formeln VIIb in Verbindungen der Formel Ia vorliegen, worin A ungleich direkte Bindung oder -CH₂CH(R')O- ist, und worin
R Methyl und insbesondere Wasserstoff ist,
R₈ Wasserstoff, Oxyl, C₁-C₁₂-Alkyl, C₁-C₁₂-Alkoxy, C₃-C₈-Alkenyl, C₃-C₈-Alkenyloxy, C₃-C₈-Alkinyl, C₅-C₆-Cycloalkyl, C₅-C₆-Cycloalkyloxy, Phenyl-C₁ -C₄-alkyl, C₁-C₈-Alkanoyl, C₃-C₅-Alkenoyl, -CH₂CH(OH)R' oder Phenoxy bedeutet,
R' Wasserstoff, Methyl oder Phenyl ist,
R₉ Wasserstoff, C₁-C₁₈-Alkyl, Allyl, C₅-C₆-Cycloalkyl, Phenyl-C₁-C₄-alkyl, durch ein oder mehrere -O-unterbrochenes C₃-C₁₈-Alkyl oder eine Gruppe der Formel IIIa 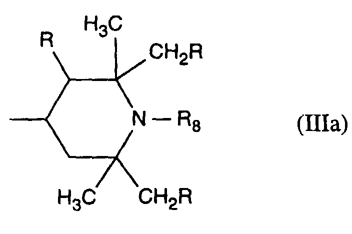 darstellt,
Q₁ die direkte Bindung, -O-C₂-C₁₂-Alkylen oder -NR₉-C₂-C₁₂-Alkylen ist, wenn A die angegebene Bedeutung haben, A aber ungleich direkte Bindung oder -CH₂CH(R')O- ist, oder in Verbindungen der Formel Ia Q₁ C₂-C₁₂-Alkylen ist, wenn A die direkte Bindung oder -CH₂CH(R')O- darstellt, oder -OCO-C₂-C₁₂-Alkylen darstellt, wenn A in Verbindungen der Formel Ia -CH₂CH(OH)CH₂- ist,
Q₂ die direkte Bindung oder -NR₉-C₂-C₁₂-Alkylen ist, wenn A ungleich direkte Bindung oder -CH₂CH(R')O- ist, oder in Verbindungen der Formel Ia Q₂ -CO-C₂-C₁₂-Alkylen darstellt, wenn A die direkte Bindung oder -CH₂CH(R')O- ist, oder -OCO-C₁-C₁₂-Alkylen oder -OCO-C₁-C₁₂-Alkylen-CO- ist, wenn A in Formel Ia -CH₂CH(OH)CH₂- ist, q 2-12, besonders 2-4 ist,
X₁ und X₂ unabhängig voneinander -O- oder -R₉N- darstellen, 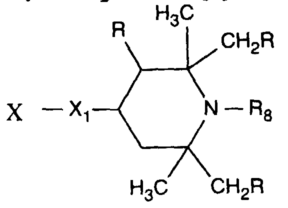 ist und Y dieselbe Bedeutung wie X hat oder C₁-C₁₈-Alkoxy oder -NR₉R₁₀ bedeutet,
R₁₀ die gleiche Bedeutung wie R₉ haben kann oder zusammen mit R₉ und dem Bindungs-N-Atom einen 5- oder 6-gliedrigen heterocyclischen Ring bildet,
Q₅ -OCH₂CH₂- oder -OCH₂CH(OH)CH₂- ist, wenn A oder B die angegebene Bedeutung haben, A aber ungleich direkte Bindung oder -CH₂CH(R')O- ist, oder in Verbindungen der Formel Ia Q₅ -CO(CH₂)₂- oder -CO(CH₂)₃- ist, wenn A die direkte Bindung oder -CH₂CH(R')O- darstellt, Q₅ die direkte Bindung bedeutet, wenn A -CH₂CH(OH)CH₂- ist, oder Q₅ -OCO(CH₂)ₛ- mit s = 1-4 ist, wenn A in Formel Ia -CH₂CH(OH)CH₂- ist,
T₁ und T₂ unabhängig voneinander Wasserstoff, C₁-C₁₈-Alkyl, Phenyl-C₁-C₄-alkyl oder unsubstituiertes oder durch Halogen oder C₁-C₄-Alkyl substituiertes Phenyl oder Naphthyl bedeuten, oder T₁ und T₂ zusammen mit dem sie verbindenden C-Atom einen C₅-C₁₂-Cycloalkanring bilden,
R₁₄ Wasserstoff, C₁-C₁₂-Alkyl, Allyl, Benzyl oder C₂-C₆-Alkoxyalkyl ist.

Hier und im folgenden bedeutet der linksstehende Bindungsstrich bei zweiwertigen Resten, wie -CH₂CH(R')O- oder -(CH₂)ₛ-CO-, die Verknüpfung zu dem linksseitig davon befindlichen Symbol oder Atom.

m und s sind bevorzugt 1. A stellt bevorzugt -CH₂CH(OH)CH₂- und insbesondere -CH₂CO- dar. R bedeutet bevorzugt Wasserstoff und R' ist insbesondere Wasserstoff oder Methyl.

Durch irgendwelche Reste dargestellte Alkyl-, Alkoxy-, Alkenyl-, Alkinyl-, Alkanoyl-, Alkenoyl-, Alkenyloxy-, Alkylen- oder Alkenylengruppen können geradkettig oder verzweigt sein. Bevorzugt sind die genannten Gruppen geradkettig.

Beispiele von Alkylgruppen R₂ bis R₆, R₈ und R₁₄ sind Methyl, Ethyl, n-Propyl, Isopropyl, n-, sek. und tert.Butyl, n-Pentyl, n-Hexyl, 2-Ethylhexyl, 2-Methylpentyl, n-Heptyl, n-Octyl, n-Nonyl, n-Decyl, n-Dodecyl; R₉, R₁₀, T₁ und T₂ können zudem als C₁-C₁₈-Alkyl n-Tetradecyl, n-Hexadecyl oder n-Octadecyl sein.

Alkylgruppen R₁ bis R₆ sind bevorzugt geradkettig und weisen 1-4 C-Atome auf. R₈ in der Bedeutung von Alkyl ist besonders C₁-C₈-Alkyl und vor allem C₁-C₄-Alkyl.

Alkylgruppen R₉, R₁₀, T₁ und T₂ weisen bevorzugt 1-12, besonders 1-8 und insbesondere 1-4 C-Atome auf. Besonders bevorzugt stellen die genannten Gruppen Ethyl und vor allem Methyl dar.

Beispiele von Alkoxygruppen R₃, R₄, R₈, und Y mit bis zu 12 bzw. 18 C-Atomen sind Methoxy, Ethoxy, n- und Isopropyloxy, n-, sek. und tert.Butyloxy, n-Pentyloxy, n-Hexyloxy, n-Heptyloxy, n-Octyloxy, n-Decyloxy, n-Dodecyloxy, n-Tetradecyloxy, n-Hexadecyloxy und n-Octadecyloxy. Bevorzugt weisen diese Alkoxygruppen 1-12 und insbesondere 1-4 C-Atome auf. Ganz besonders bevorzugt sind Ethoxy- und insbesondere Methoxygruppen.

Stellen irgendwelche Substituenten Alkylengruppen dar oder enthalten diese Substituenten Alkylenteile, so handelt es sich dabei bevorzugt um geradkettiges Alkylen mit vorzugsweise 2 bis 12 C-Atomen.

Enthalten etwaige Substituenten Phenylen-, Naphthylen- oder Biphenylengruppen, so handelt es sich z.B. um o-, m- oder p-Phenylen, 1,4-Naphthylen oder 4,4'-Biphenylen.

Stellen R₁ bis R₆ Halogen dar, so handelt es sich insbesondere um Chlor oder Brom.

R₁ und R₂, R₃ und R₄ sowie R₅ und R₆ haben bevorzugt je paarweise dieselbe Bedeutung. Vorzugsweise sind R₁ und R₂ sowie R₃ und R₄ je paarweise Wasserstoff oder C₁-C₄-Alkyl und R₅ und R₆ sind je Wasserstoff. In Formel Ia stellen R₁ und R₂ bzw. R₃ und R₄ insbesondere je Wasserstoff, -OH oder Methyl dar.

R₅ und R₆ sind bevorzugt Wasserstoff Besonders bevorzugt stellen R₁ und R₂ in Formel I bzw. Ia je Wasserstoff oder Methyl dar und R₃ und R₄ sind vorzugsweise je Wasserstoff und insbesondere je Methyl.

R₈ als Alkyl oder Alkoxy weist vorzugsweise 1-8 und insbesondere 1-4 C-Atome auf. Bedeutet R₈ C₃-C₈-Alkenyl oder C₃-C₈-Alkenyloxy, so handelt es sich z.B. um 1-Propenyl, Allyl, Methallyl, 2-Butenyl, 2-Pentenyl, 2-Hexenyl, 2-Octenyl oder 4-tert.Butyl-2-butenyl oder die entsprechenden Alkenyloxyreste. R₅ als C₃-C₈-Alkinyl ist bevorzugt Propargyl. C₅-C₆-Cycloalkyl- oder C₅-C₆-Cycloalkoxygruppen R₈ sind insbesondere Cyclohexyl oder Cyclohexyloxy. Stellt R₈ C₁-C₈-Alkanoyl dar, so handelt es sich z.B. um Formyl, Propionyl, Isobutyryl, Hexanoyl oder Octanoyl, bevorzugt um Acetyl. C₃-C₉-Alkenoyl ist vorzugsweise Acryloyl oder Methacryloyl. Bedeutet R₈, R₉ oder R₁₀ Phenyl-C₁-C₄-alkyl, so kommen insbesondere Phenylethyl und vor allem Benzyl in Betracht.

R₈ ist bevorzugt Wasserstoff, Oxyl, C₁-C₁₂-Alkyl, C₃-C₈-Alkenyl, C₃-C₈-Alkinyl, Phenyl-C₁-C₄-alkyl, C₁-C₈-Alkanoyl, C₃-C₈-Alkenoyl oder -CH₂CH(OH)R'. Besonders bevorzugt stellt R₈ Wasserstoff, Oxyl, C₁-C₈-, besonders C₁-C₄-Alkyl, Allyl, Benzyl, Acetyl oder Acryloyl und insbesondere Wasserstoff, Methyl, Allyl, Benzyl oder Acetyl dar. Ganz besonders bevorzugt sind Wasserstoff und Methyl.

Alkylgruppen R₉ und R₁₀ weisen bevorzugt 1-12, insbesondere 1-8 und vor allem 1-4 C-Atome auf. Ganz besonders bevorzugt sind Ethyl und vor allem Methyl. Bedeuten R₉ oder R₁₀ C₅-C₆-Cycloalkyl, so handelt es sich insbesondere um Cyclohexyl. Als C₃-C₁₈-Alkyl, das durch ein oder mehrere O-Atome unterbrochen ist, bedeuten R₉ und R₁₀ z.B. -CH₂CH₂OCH₃, -CH₂OCH₂CH₃, -CH₂CH₂OC₄H₉ oder -CH₂CH₂OCH₂CH₂CH₃.

R₉ und R₁₀ stellen vorzugsweise unabhängig voneinander Wasserstoff, C₁-C₈- und insbesondere C₁-C₄-Alkyl, vor allem Ethyl oder Methyl, Allyl, Cyclohexyl, Benzyl oder eine Gruppe der Formel IIIa dar, worin R Wasserstoff ist und R₅ die oben erwähnte bevorzugte Bedeutung hat. Besonders bevorzugt sind R₉ und R₁₀ je H oder C₁-C₄-Alkyl, ganz besonders bevorzugt je Wasserstoff.

Als Reste der Formel V sind solche bevorzugt, worin R Wasserstoff ist, Q₁ -NH-C₂-C₄-Alkylen oder -O-C₂-C₄-Alkylen und insbesondere die direkte Bindung bedeutet und R₈ die oben angegebene bevorzugte Bedeutung hat. Beispiele für Verbindungen der Formel Ia mit Resten der Formel V sind: 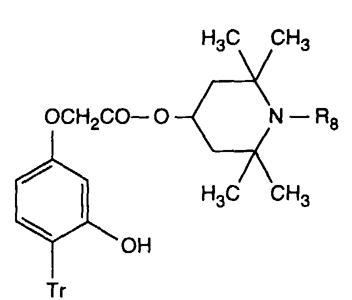 mit R₈ = H, Methyl, Benzyl, Allyl oder Acetyl 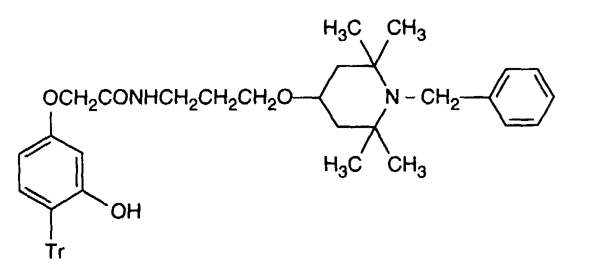 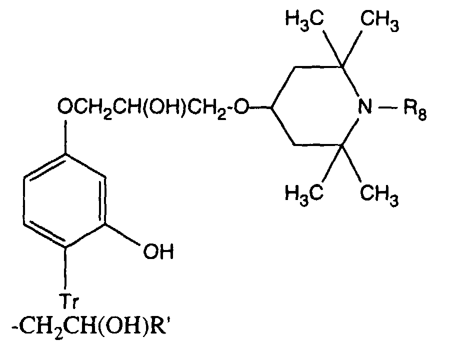 R₈ = H, -CH₃, Benzyl, Allyl, -COCH₃,

In bevorzugten Resten der Formel VI stellt R Wasserstoff dar, R₉ ist vorzugsweise C₁-C₈-Alkyl, insbesondere C₁-C₄-Alkyl und vor allem Wasserstoff, Q₂ ist die direkte Bindung oder -NR₉-C₂-C₄-Alkylen oder -OCO-C₁-C₄-Alkylen oder -OCO-C₂-C₄-Alkylen-CO- (A = -CH₂CH(OH)CH₂-) und R₈ hat die oben angegebene bevorzugte Bedeutung. Beispiele von Verbindungen der Formel Ia derartigen Resten sind:

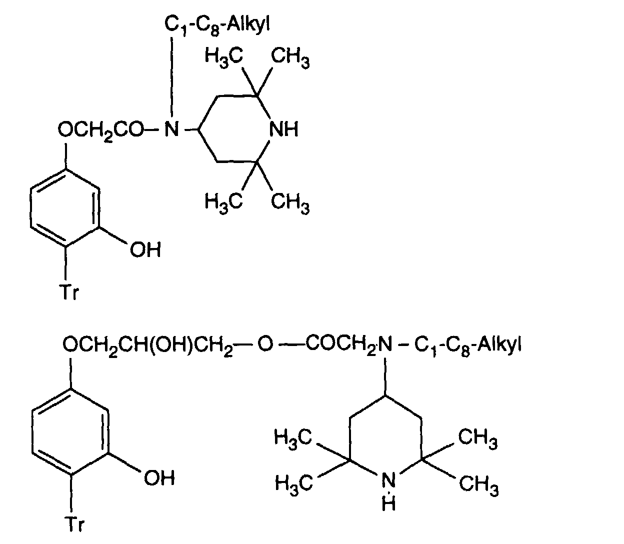

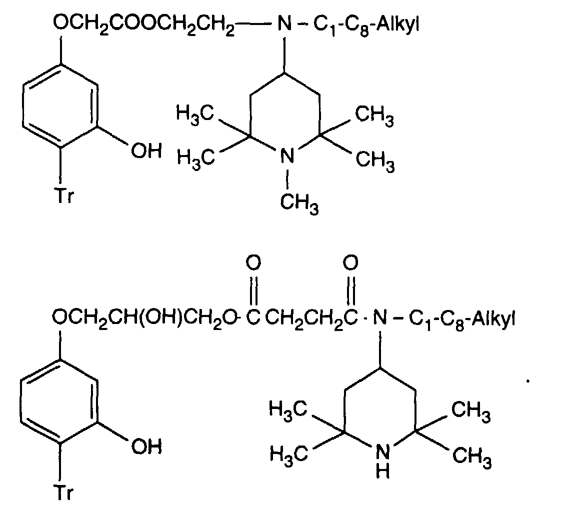

X in Resten der Formel VIIa und VIIb stellt bevorzugt 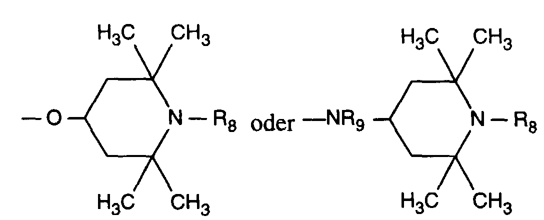 mit R₉ = H oder C₁-C₄-Alkyl dar, wobei R₈ die oben angegebene bevorzugte Bedeutung hat.

Y hat vorzugsweise dieselbe Bedeutung wie X oder bedeutet C₁-C₈-, insbesondere C₁-C₄-Alkoxy, -NR₉R₁₀ mit R₉ und R₁₀ = unabhängig voneinander H, C₁-C₈- und insbesondere C₁-C₄-Alkyl, Allyl, Cyclohexyl oder Benzyl. Bilden R₉ und R₁₀ zusammen mit dem Bindungs-C-Atom einen 5- oder 6-gliedrigen Ring, so handelt es sich z.B. um einen Pyrrolidin-, Piperidin-, 2,6-Dimethylpiperidin-, Piperazin-, 4-Methylpiperazin- oder Morpholinring. X₁ und X₂ in Formel VIIb sind bevorzugt unabhängig voneinander -O-oder -NH-.

Als Beispiel von Verbindungen mit solchen Triazinresten seien erwähnt: 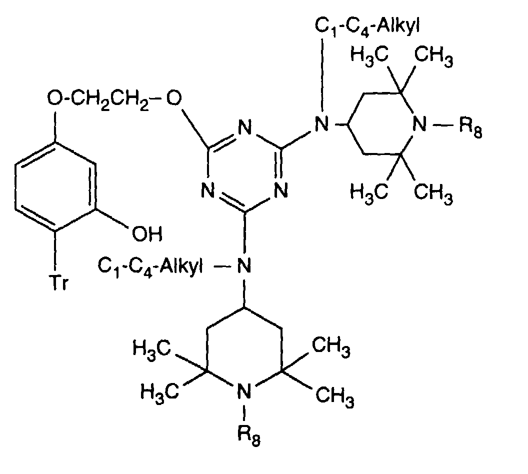
R₈ = oben angegebene bevorzugte Bedeutung, insbesondere H oder Methyl.

In Resten der Formel Xa oder Xb ist R bevorzugt Wasserstoff und R₈ hat die oben angegebene bevorzugte Bedeutung. R₁₄ als C₂-C₆-Alkoxyalkyl ist z.B. Methoxymethyl, Ethoxymethyl, Propoxymethyl, tert.Butoxymethyl, Ethoxyethyl, Ethoxypropyl oder Propoxypropyl. Vorzugsweise stellt R₁₄ C₁-C₄-Alkyl, Allyl oder Benzyl und insbesondere H dar. Alkylgruppen T₁ und T₂ sind bevorzugt geradkettig und weisen insbesondere 1-4 C-Atome auf Als Phenyl-C₁-C₄-alkyl sind T₁ und T₂ insbesondere Phenylethyl und vor allem Benzyl. Bilden T₁ und T₂ zusammen mit dem Bindungs-C-Atom einen Cycloalkanring, so kann dies z.B. ein Cyclopentan-, Cyclohexan-, Cyclooctan- oder Cyclododecanring sein. Vorzugsweise stellen T₁ und T₂ Wasserstoff dar oder sie bilden zusammen mit dem Bindungs-C-Atom einen Cyclododecanring.

Als Beispiele von Verbindungen der Formel Ia mit solchen Resten seien genannt: 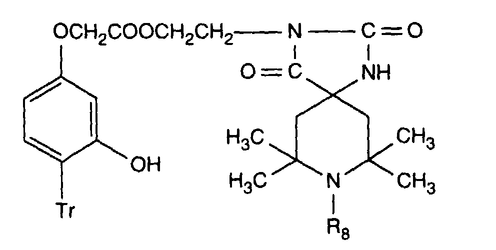 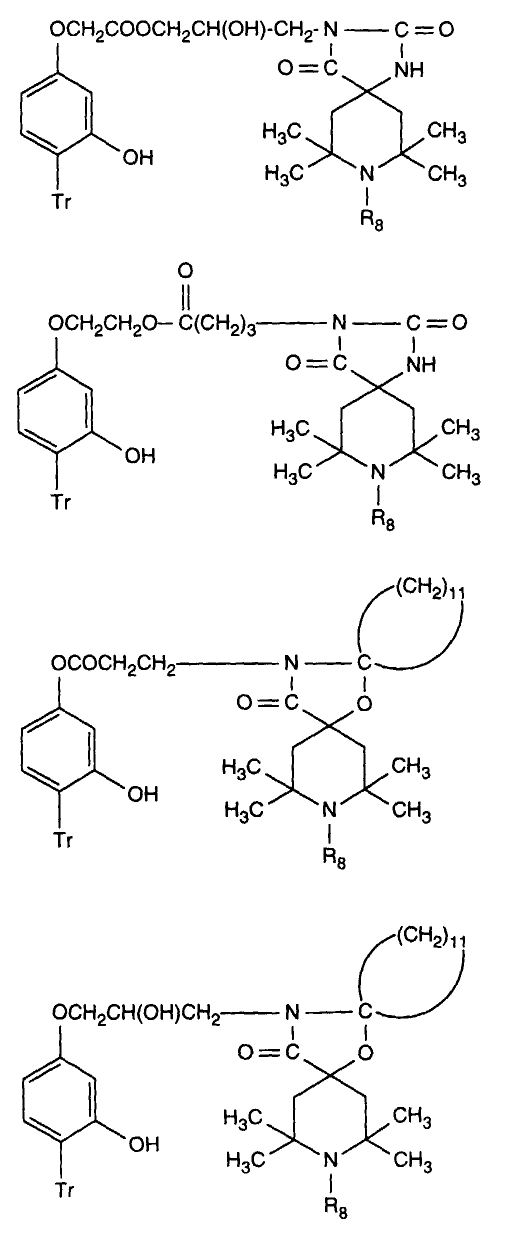
mit R₈ = H oder Methyl und
R₃ = R₄ = H oder C₁-C₈-Alkoxy.

Bevorzugt sind Verbindungen der Formel Ia, worin A ist, R₁ und R₂ sowie R₃ und R₄ je paarweise Wasserstoff oder C₁-C₄-Alkyl darstellen, R₅ und R₆ je Wasserstoff sind, R₇ bzw. R₇' einen Rest der Formel V darstellen, Q₁ die direkte Bindung ist, R₈ Wasserstoff, C₁-C₄-Alkyl, Benzyl, Allyl, Acetyl oder Acryloyl bedeutet, und R' Wasserstoff, Methyl oder Phenyl darstellt.

Ganz besonders bevorzugt sind Verbindungen der Formel Ia, worin A -CH₂CO- ist, R₁ und R₂ je Wasserstoff und insbesondere je Methyl darstellen, R₃ und R₄ je Methyl und R₅ und R₆ je Wasserstoff sind und R₇ einen Rest der Formel V bedeutet, worin Q₁ die direkte Bindung, R₈ Wasserstoff, Methyl, Benzyl, Allyl oder Acetyl, und R' Wasserstoff oder Methyl darstellen.

Des weiteren bevorzugt sind Verbindungen der Formel Ia, worin A -CH₂CH(OH)CH₂- ist, R₁ und R₂ sowie R₃ und R₄ je paarweise Wasserstoff oder C₁-C₄-Alkyl darstellen, R₅ und R₆ je Wasserstoff sind, R₇ einen Rest der Formel V darstellt, Q₁ die direkte Bindung ist, R₈ Wasserstoff, C₁-C₄-Alkyl, Benzyl, Allyl, Acetyl oder -CH₂CH(OH)R' ist, insbesondere solche, worin A -CH₂CH(OH)CH₂- ist, R₁ und R₂ je Wasserstoff und insbesondere je Methyl darstellen, R₃ und R₄ je Methyl und R₅ und R₆ je Wasserstoff sind, R₇ einen Rest der Formel V darstellt, Q₁ die direkte Bindung ist, R₈ Methyl, Benzyl, Allyl oder -CH₂CH(OH)CH₃ ist.

Die erfindungsgemässen Verbindungen können nach an sich bekannten Methoden durch Umsetzung von Gruppierungen der Formel I oder II und Gruppierungen der Formel III enthaltenden funktionellen Derivaten hergestellt werden;

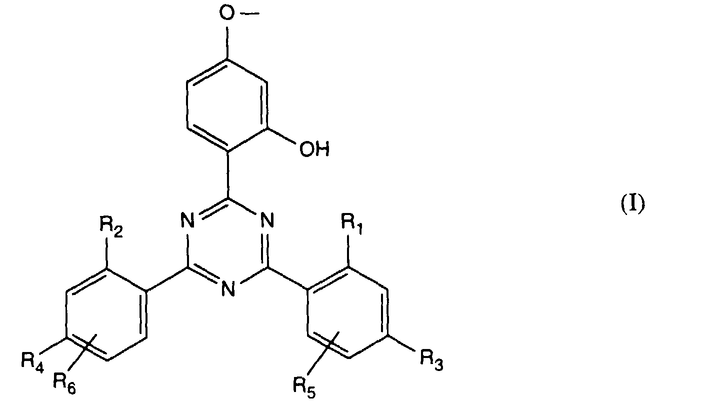

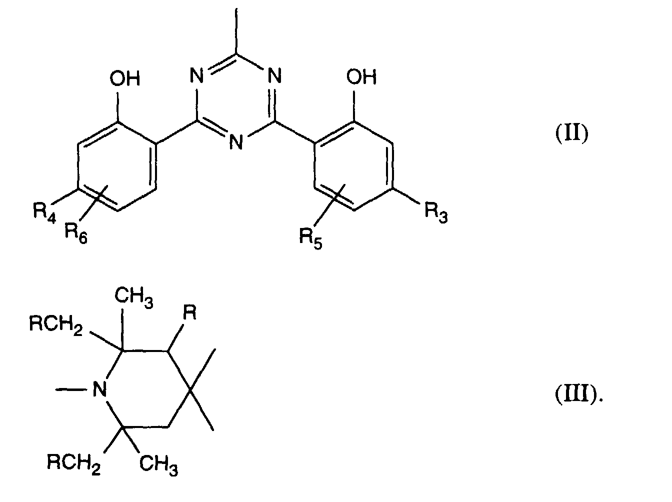

Stellt mindestens eines von R₃ bis R₆ einen eine Gruppierung der Formel III enthaltenden Rest dar, so können Reste der Formel I oder II enthaltende funktionelle Derivate auch mit an sich beliebigen, keine Polyalkylpiperidinreste aufweisenden reaktionsfähigen Verbindungen umgesetzt werden.
a) Verbindungen der Formel Ia, worin A -(CH₂)ₘCO- bedeutet, können z.B. durch Umsetzung von Carbonsäureestern der Formel XX 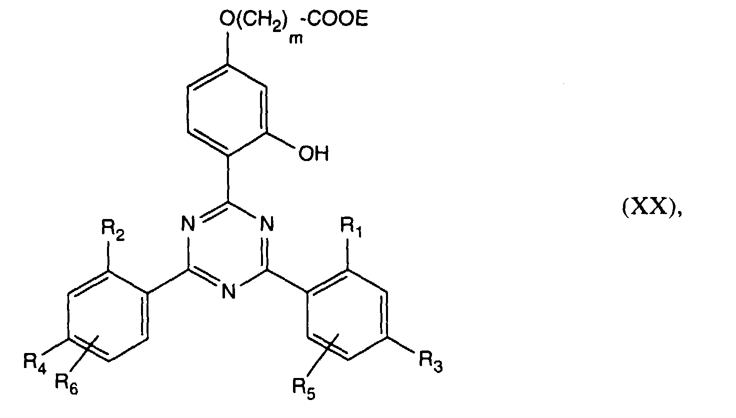 worin E z.B. C₁-C₄-Alkyl darstellt, mit geeigneten, Reste R₇ enthaltenden funktionellen Derivaten, wie Alkoholen oder Aminen, erhalten werden. Die Ausgangsprodukte der Formel XX können ebenfalls auf konventionelle Weise durch Umsetzung der entsprechenden o-Hydroxyphenyl-1,3,5-triazine der Formel XXI 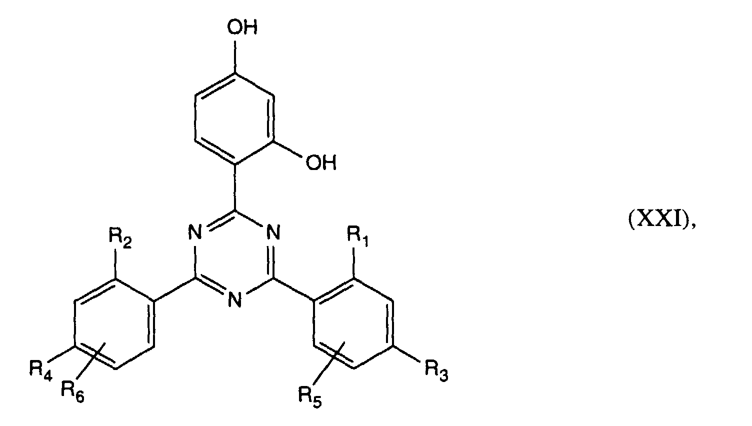 mit Halogencarbonsäureestern Hal(CH₂)ₘ-COOE in alkalischem Medium erhalten werden; vgl. CH-A 484 695. Die Verbindungen der Formel XXI sind bekannte Verbindungen und können durch Friedel-Crafts-Reaktion von Cyanurchlorid mit je 1 Mol einer Verbindung der Formel 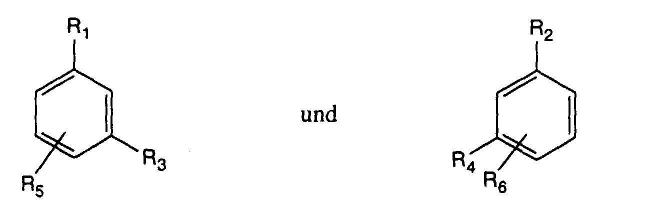 und 1 Mol Resorcin hergestellt werden, wie dies z.B. in CH-A 480 091 und CH-A 484 695 beschrieben ist.
   Gruppierungen der Formel III enthaltende 4-Hydroxy-, 4-Amino- oder in 1-Stellung unsubstituierte Piperidinderivate können auch zuerst mit den genannten Halogencarbonsäureestern umgesetzt werden, worauf man diese in alkalischem Medium mit den o-Hydroxyphenyltriazinen der Formel XXI reagieren lässt.
b) Verbindungen der Formel Ia mit A = -CH₂CH(R')-O- können durch Umsetzung der o-Hydroxyphenyltriazine der Formel XXI mit Epoxiden 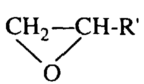 und geeigneten Polyalkylpiperidinderivaten, wie Estern, und insbesondere mit Gruppen der Formel III enthaltenden Chlortriazinen erhalten werden.
   Verbindungen der Formel Ia mit A = -CH₂CH(R)-O- können auch dadurch hergestellt werden, dass man ein o-Hydroxyphenyltriazin der Formel XXI, ein Dihalogenid Hal-CH₂CH(R')-Hal, bevorzugt mit Halogenatomen unterschiedlicher Reaktivität, wie z.B. 1-Brom-2-chlorethan, und ein mindestens eine Gruppe der Formel III enthaltendes Mono- oder Dihydroxypiperidinderivat in an sich beliebiger Reihenfolge miteinander reagieren lässt.
c) Verbindungen der Formel Ia mit A = -CH₂CH(OH)CH₂- erhält man z.B. dadurch, dass man ein o-Hydroxyphenyltriazin der Formel XXI, das entsprechende Epoxid und mindestens eine Gruppe der Formel III enthaltende Ester, Alkohole oder Amine in an sich beliebiger Reihenfolge miteinander reagieren lässt.
d) Verbindungen der Formel Ia, worin A eine direkte Bindung darstellt, lassen sich z.B. durch Umsetzung von o-Hydroxyphenyltriazinen der Formel XXI mit mindestens eine Gruppierung der Formel III enthaltenden Estern oder Halogeniden herstellen.

Die für die obigen Umsetzungen benötigten funktionellen Polyalkylpiperidinderivate sind an sich bekannt oder können nach bekannten Methoden hergestellt werden.

Weisen irgendwelche Substituenten am N-Atom des Piperidinringes funktionelle Gruppen auf, so können diese auf an sich bekannte Weise nach der Umsetzung der Triazin- mit den Piperidinderivaten eingeführt werden, z.B. durch Hydroxyalkylierung.

Die erfindungsgemässen Verbindungen sind verwendbar als Stabilisatoren für organische Materialien gegen Schädigung durch Licht, Sauerstoff oder Hitze. Ganz besonders eignen sich die erfindungsgemässen Verbindungen als Lichtstabilisatoren. Solche zu stabilisierenden Materialien können z.B. Oele, Fette, Wachse, Kosmetika, Biocide oder photographische Materialien sein. Von besonderem Interesse ist die Verwendung in polymeren Materialien, wie sie in Kunststoffen, Kautschuken, Anstrichstoffen oder Klebstoffen vorliegen. Beispiele für Polymere und andere Substrate, die auf diese Weise stabilisiert werden können, sind die folgenden:
1. Polymere von Mono- und Diolefinen, beispielsweise Polypropylen, Polyisobutylen, Polybuten-1, Polymethylpenten-1, Polyisopren oder Polybutadien sowie Polymerisate von Cycloolefinen wie z.B. von Cyclopenten oder Norbornen; ferner Polyethylen (das gegebenenfalls vernetzt sein kann), z.B. Polyethylen hoher Dichte (HDPE), Polyethylen niederer Dichte (LDPE), lineares Polyethylen niederer Dichte (LLDPE).
2. Mischungen der unter 1) genannten Polymeren, z.B. Mischungen von Polypropylen mit Polyisobutylen, Polypropylen mit Polyethylen (z.B. PP/HDPE, PP/LDPE) und Mischungen verschiedener Polyethylentypen (z.B. LDPE/HDPE).
3. Copolymere von Mono- und Diolefinen untereinander oder mit anderen Vinylmonomeren, wie z.B. Ethylen-Propylen-Copolymere, lineares Polyethylen niederer Dichte (LLDPE) und Mischungen desselben mit Polyethylen niederer Dichte (LDPE), Propylen-Buten-1-Copolymere, Propylen-Isobutylen-Copolymere, Ethylen-Buten-1-Copolymere, Ethylen-Hexen-Copolymere, Ethylen-Methylpenten-Copolymere, Ethylen-Hepten-Copolymere, Ethylen-Octen-Copolymere, Propylen-Butadien-Copolymere, Isobutylen-Isopren-Copolymere, Ethylen-Alkylacrylat-Copolymere, Ethylen-Alkylmethacrylat- Copolymere, Ethylen-Vinylacetat-Copolymere oder Ethylen-Acrylsäure-Copolymere und deren Salze (Ionomere), sowie Terpolymere von Ethylen mit Propylen und einem Dien, wie Hexadien, Dicyclopentadien oder Ethylidennorbornen; ferner Mischungen solcher Copolymere untereinander und mit unter 1) genannten Polymeren, z.B. Polypropyten/Ethyten-Propylen-Copolymere, LDPE/Ethylen-Vinylacetat-Copolymere, LDPE/Ethylen-Acrylsäure-Copolymere, LLDPE/Ethylen-Vinylacetat-Copolymere und LLDPE/Ethylen-Acrylsäure-Copolymere.
3a. Kohlenwasserstoffharze (z.B. C₅-C₉) inklusive hydrierte Modifikationen davon (z.B. Klebrigmacherharze).
4. Polystyrol, Poly-(p-methylstyrol), Poly-(α-methylstyrol).
5. Copolymere von Styrol oder α-Methylstyrol mit Dienen oder Acrylderivaten, wie z.B. Styrol-Butadien, Styrol-Acrylnitril, Styrol-Alkylmethacrylat, Styrol-Butadien-Alkylacrylat, Styrol-Maleinsäureanhydrid, Styrol-Acrylnitril-Methylacrylat; Mischungen von hoher Schlagzähigkeit aus Styrol-Copolymeren und einem anderen Polymer, wie z.B. einem Polyacrylat, einem Dien-Polymeren oder einem Ethylen-Propylen-Dien-Terpolymeren; sowie Block-Copolymere des Styrols, wie z.B. Styrol-Butadien-Styrol, Sryrol-Isopren-Styrol, Styrol-Ethylen/Butylen-Styrol oder Styrol-Ethylen/Propylen-Styrol.
6. Pfropfcopolymere von Styrol oder α-Methylstyrol, wie z.B. Styrol auf Polybutadien, Styrol auf Polybutadien-Styrol- oder Polybutadien-Acrylnitril-Copolymere, Styrol und Acrylnitril (bzw. Methacrylnitril) auf Polybutadien; Styrol, Acrylnitril und Methylmethacrylat auf Polybutadien; Styrol und Maleinsäureanhydrid auf Polybutadien; Styrol, Acrylnitril und Maleinsäureanhydrid oder Maleinsäureimid auf Polybutadien; Styrol und Maleinsäureimid auf Polybutadien, Styrol und Alkylacrylate bzw. Alkylmethacrylate auf Polybutadien, Styrol und Acrylnitril auf Ethylen-Propylen-Dien-Terpolymeren, Styrol und Acrylnitril auf Polyalkylacrylaten oder Polyalkylmethacrylaten, Styrol und Acrylnitril auf Acrylat-Butadien-Copolymeren, sowie deren Mischungen mit den unter 5) genannten Copolymeren, wie sie z.B. als sogenannte ABS-, MBS-, ASA- oder AES-Polymere bekannt sind.
7. Halogenhaltige Polymere, wie z.B. Polychloropren, Chlorkautschuk, chloriertes oder chlorsulfoniertes Polyethylen, Copolymere von Ethylen und chloriertem Ethylen, Epichlorhydrinhomo- und -copolymere, insbesondere Polymere aus halogenhaltigen Vinylverbindungen, wie z.B. Polyvinylchlorid, Polyvinylidenchlorid, Polyvinylfluorid, Polyvinylidenfluorid; sowie deren Copolymere, wie Vinylchlorid-Vinylidenchlorid, Vinylchlorid-Vinylacetat oder Vinylidenchlorid-Vinylacetat.
8. Polymere, die sich von α,β-ungesättigten Säuren und deren Derivaten ableiten, wie Polyacrylate und Polymethacrylate, Polyacrylamide und Polyacrylnitrile.
9. Copolymere der unter 8) genannten Monomeren untereinander oder mit anderen ungesättigten Monomeren, wie z.B. Acrylnitril-Butadien-Copolymere, Acrylnitril-Alkylacrylat-Copolymere, Acrylnitril-Alkoxyalkylacrylat-Copolymere, Acrylnitril-Vinylhalogenid-Copolymere oder Acrylnitril-Alkylmethacrylat-Butadien-Terpolymere.
10. Polymere, die sich von ungesättigten Alkoholen und Aminen bzw. deren Acylderivaten oder Acetalen ableiten, wie Polyvinylalkohol, Polyvinylacetat, -stearat, -benzoat, -maleat, Polyvinylbutyral, Polyallylphthalat, Polyallylmelamin; sowie deren Copolymere mit in Punkt 1 genannten Olefinen.
11. Homo- und Copolymere von cyclischen Ethern, wie Polyalkylenglykole, Polyethylenoxyd, Polypropylenoxyd oder deren Copolymere mit Bisglycidylethern.
12. Polyacetale, wie Polyoxymethylen, sowie solche Polyoxymethylene, die Comonomere, wie z.B. Ethylenoxid, enthalten; Polyacetale, die mit thermoplastischen Polyurethanen, Acrylaten oder MBS modifiziert sind.
13. Polyphenylenoxide und -sulfide und deren Mischungen mit Styrolpolymeren oder Polyamiden.
14. Polyurethane, die sich von Polyethern, Polyestern und Polybutadienen mit endständigen Hydroxylgruppen einerseits und aliphatischen oder aromatischen Polyisocyanaten andererseits ableiten, sowie deren Vorprodukte.
15. Polyamide und Copolyamide, die sich von Diaminen und Dicarbonsäuren und/oder von Aminocarbonsäuren oder den entsprechenden Lactamen ableiten, wie Polyamid 4, Polyamid 6, Polyamid 6/6, 6/10, 6/9, 6/12, 4/6, Polyamid 11, Polyamid 12, aromatische Polyamide ausgehend von m-Xylylen-diamin und Adipinsäure; Polyamide, hergestellt aus Hexamethylendiamin und Iso- und/oder Terephthalsäure und gegebenenfalls einem Elastomer als Modifikator, z.B. Poly-2,4,4-trimethylhexamethylenterephthalamid, Poly-m-phenylen-isophthalamid. Block-Copolymere der vorstehend genanntenPolyamide mit Polyolefinen, Olefin-Copolymeren, Ionomeren oder chemisch gebundenen oder gepfropften Elastomeren; oder mit Polyethern, wie z.B. mit Polyethylenglykol, Polypropylenglykol oder Polytetramethylenglykol. Ferner mit EPDM oder ABS modifizierte Polyamide oder Copolyamide; sowie während der Verarbeitung kondensierte Polyamide ("RIM-Polyamidsysteme").
16. Polyharnstoffe, Polyimide, Polyamid-imide und Polybenzimidazole.
17. Polyester, die sich von Dicarbonsäuren und Dialkoholen und/oder von Hydroxycarbonsäuren oder den entsprechenden Lactonen ableiten, wie Polyethylenterephthalat, Polybutylenterephthalat, Poly-1,4-dimethylolcyclohexanterephthalat, Polyhydroxybenzoate, sowie Block-Polyether-ester, die sich von Polyethern mit Hydroxylendgruppen ableiten; ferner mit Polycarbonaten oder MBS modifizierte Polyester.
18. Polycarbonate und Polyestercarbonate.
19. Polysulfone, Polyethersulfone und Polyetherkerone.
20. Vernetzte Polymere, die sich von Aldehyden einerseits und Phenolen, Harnstoff oder Melamin andererseits ableiten, wie Phenol-Formaldehyd-, Harnstoff-Formaldehyd- und Melamin-Formaldehydharze.
21. Trocknende und nicht-trocknende Alkydharze.
22. Ungesättigte Polyesterharze, die sich von Copolyestern gesättigter und ungesättigter Dicarbonsäuren mit mehrwertigen Alkoholen, sowie Vinylverbindungen als Vernetzungsmittel ableiten, wie auch deren halogenhaltige, schwerbrennbare Modifikationen.
23. Vernetzbare Acrylharze, die sich von substituierten Acrylsäureestern ableiten, wie z.B. von Epoxyacrylaten, Urerhan-acrylaten oder Polyester-acrylaten.
24. Alkydharze, Polyesterharze und Acrylatharze, die mit Melaminharzen, Harnstoffharzen, Polyisocyanaten oder Epoxidharzen vernetzt sind.
25. Vernetzte Epoxidharze, die sich von Polyepoxiden ableiten, z.B. von Bis-glycidylethern oder von cycloaliphatischen Diepoxiden.
26. Natürliche Polymere, wie Cellulose, Naturkautschuk, Gelatine, sowie deren polymerhomolog chemisch abgewandelte Derivate, wie Celluloseacetate, -propionate und -butyrate, bzw. die Celluloseether, wie Methylcellulose; sowie Kolophoniumharze und Derivate.
27. Mischungen (Polyblends) der vorgenannten Polymeren, wie z.B. PP/EPDM, Polyamid/EPDM oder ABS, PVC/EVA, PVC/ABS, PVC/MBS, PC/ABS, PBTP/ABS, PC/ASA, PC/PBT, PVC/CPE, PVC/Acrylate, POM/thermoplastisches PUR, PC/thermoplastisches PUR, POM/Acrylat, POM/MBS, PPO/HIPS, PPO/PA 6.6 und Copolymere, PA/HDPE, PA/PP, PA/PPO.
28. Natürliche und synthetische organische Stoffe, die reine monomere Verbindungen oder Mischungen von solchen darstellen, beispielsweise Mineralöle, tierische oder pflanzliche Fette, Oele und Wachse, oder Oele, Wachse und Fette auf Basis synthetischer Ester (z.B. Phthalate, Adipate, Phosphate oder Trimellitate), sowie Abmischungen synthetischer Ester mit Mineralölen in beliebigen Gewichtsverhältnissen, wie sie z.B. als Spinnpräparationen Anwendung finden, sowie deren wässrige Emulsionen.
29. Wässrige Emulsionen natürlicher oder synthetischer Kautschuke, wie z.B. Naturkautschuk-Latex oder Latices von carboxylierten Styrol-Butadien-Copolymeren.

Gegenstand der Erfindung ist somit auch ein gegen Licht, Sauerstoff oder/und Hitze empfindliches organisches Material, das mindestens eine erfindungsgemässe Verbindung enthält. Als organische Materialien sind insbesondere synthetische Polymere zu erwähnen, z.B. thermoplastische Kunststoffe oder Elastomere, insbesondere erstere. Besonders hervorzuheben sind Polyolefine als organische Materialien.

Besonders bevorzugt ist die Verwendung der erfindungsgemässen Verbindungen in Lacken aller Art. Dies können pigmentierte oder unpigmentierte Lacke oder Metalleffektlacke sein. Sie können ein organisches Lösungsmittel enthalten oder lösungsmittelfrei sein oder wässrige Lacke sein.

Die Lacke können als Bindemittel mindestens eines der vorhin aufgeführten Polymeren enthalten. Beispiele für Lacke mit speziellen Bindemitteln sind die folgenden:
1. Lacke auf Basis von kalt- oder heiss-vernetzbaren Alkyd-, Acrylat-, Polyester-, Epoxid- oder Melaminharzen oder Mischungen solcher Harze, gegebenenfalls mit Zusatz eines sauren Härtungskatalysators;
2. Zweikomponenten-Polyurethanlacke auf Basis von hydroxylgruppenhaltigen Acrylat-, Polyester- oder Polyetherharzen und aliphatischen oder aromatischen Polyisocyanaten;
3. Einkomponenten-Polynrethanlacke auf Basis von blockierten Polyisocyanaten, die während des Einbrennens deblockiert werden;
4. Zweikomponentenlacke auf Basis von (Poly)ketiminen und aliphatischen oder aromatischen Polyisocyanaten;
5. Zweikomponentenlacke auf Basis von (Poly)ketiminen und einem ungesättigten Acrylatharz oder einem Polyacetoacetatharz oder einem Methylacrylamidoglykolatmethylester;
6. Zweikomponentenlacke auf Basis von carboxyl- oder aminogruppenhaltigen Polyacrylaten und Polyepoxiden;
7. Zweikomponentenlacke auf Basis von anhydridgruppenhaltigen Acrylatharzen und einer Polyhydroxy- oder Polyaminokomponente;
8. Zweikomponentenlacke auf Basis von (Poly)oxazolidinen und anhydridgruppenhaltigen Acrylatharzen oder ungesättigten Acrylatharzen oder aliphatischen oder aromatischen Polyisocyanaten.
9. Zweikomponentenlacke auf Basis von ungesättigten Polyacrylaten und Polymalonaten;
10. thermoplastische Polyacrylatlacke auf Basis von thermoplastischen Acrylatharzen oder fremdvernetzenden Acrylatharzen in Kombination mit veretherten Melaminharzen;
11. Lacksysteme auf Basis von siloxanmodifizierten Acrylatharzen;
12. Lacksysteme auf Basis von fluormodifizierten Acrylatharzen, und
13. Lacksysteme auf Basis von Allylglycidylethern.

Die Lacke können auch strahlenhärtbare Lacke sein. In diesem Fall besteht das Bindungsmittel aus monomeren oder oligomeren Verbindungen, die ethylenische Doppelbindungen enthalten und durch Bestrahlung mit aktinischem Licht oder mit Elektronenstrahlen in eine vernetzte hochmolekulare Form übergehen. Meist handelt es sich hierbei um ein Gemisch solcher Verbindungen.

Die Lacke können als Einschicht- oder Zweischichtlacke appliziert werden, wobei die erfindungsgemässen Stabilisatoren vorzugsweise der unpigmentierten obersten Schicht zugesetzt werden.

Die Lacke können auf die Substrate (Metall, Plastik, Holz etc.) nach den üblichen Verfahren aufgebracht werden, beispielsweise durch Streichen, Besprühen, Giessen, Tauchen oder Elektrophorese.

Eine bevorzugte Ausführungsform der vorliegenden Erfindung sind somit Anstrichstoffe bzw. Lacke (z.B. Automobillacke), die mindestens eine erfindungsgemässe Verbindung enthalten. Als Bindemittel kommen beispielsweise die vorstehend genannten in Betracht.

Die Menge des Zusatzes an erfindungsgemässen Verbindungen hängt vom Substrat und den Anforderungen an dessen Stabilität ab. Im allgemeinen setzt man, bezogen auf das organische Material, 0,01 bis 10 Gew.%, insbesondere 0,02 bis 5 Gew.%, vor allem 0,05-3, z.B. 0,1-2 Gew.% davon zu. Es können auch Gemische verschiedener definitionsgemässer Verbindungen zugesetzt werden.

Die Einarbeitung in die organischen Materialien kann beispielsweise durch Einmischen der erfindungsgemässen Verbindungen und gegebenenfalls weiterer Additive nach den in der Technik üblichen Methoden erfolgen. Handelt es sich um Polymere, insbesondere synthetische Polymere, kann die Einarbeitung vor oder während der Formgebung, oder durch Aufbringen der gelösten oder dispergierten Verbindungen auf das Polymere, gegebenenfalls unter nachträglichem Verdunsten des Lösungsmittels erfolgen. Im Fall von Elastomeren können diese auch als Latices stabilisiert werden. Eine weitere Möglichkeit der Einarbeitung der erfindungsgemässen Verbindungen in Polymere besteht in deren Zugabe vor oder während der Polymerisation der entsprechenden Monomeren bzw. vor der Vernetzung.

Die erfindungsgemässen Verbindungen oder Mischungen davon können auch in Form eines Masterbatches, der diese Verbindungen beispielsweise in einer Konzentration von 2,5 bis 25 Gew.% enthält, den zu stabilisierenden Kunststoffen zugesetzt werden.

Zweckmässig kann die Einarbeitung der erfindungsgemässen Verbindungen nach folgenden Methoden erfolgen:
- als Emulsion oder Dispersion (z.B. zu Latices oder Emulsionspolymeren)
- als Trockenmischung während des Vermischens von Zusatzkomponenten oder Polymermischungen
- durch direktes Zugeben in die Verarbeitungsapparatur (z.B. Extruder, Innenmischer usw.)
- als Lösung oder Schmelze.

Die Einarbeitung in Lacke bzw. Lackzusammensetzungen erfolgt ebenfalls nach üblichen Methoden. Zweckmässig werden 0,01 bis 5, insbesondere 0,02 bis 3 Gew.% an erfindungsgemässer Verbindung zugesetzt. Die Zugabe zur Lackformulierung kann vor, gemeinsam mit oder nach Zugabe des Bindemittels und gegebenenfalls weiterer Komponenten erfolgen.

Ausser den erfindungsgemässen Stabilisatoren können dem Polymeren noch andere Stabilisatoren zugesetzt werden. Beispiele hierfür sind:
1. Antioxidantien
   1.1. Alkylierte Monophenole, z.B. 2,6-Di-tert.butyl-4-methylphenol, 2-Tert.butyl-4,6-di-methylphenol, 2,6-Di-tert.butyl-4-ethylphenol, 2,6-Di-tert.butyl-4-n-butylphenol, 2,6-Di-tert.butyl-4-i-butylphenol, 2,6-Di-cyclopentyl-4-methylphenol, 2-(α-Methylcyclohexyl)-4,6-dimethylphenol, 2,6-Di-octadecyl-4-methylphenol, 2,4,6-Tri-cyclohexylphenol, 2,6-Di-tert.butyl-4-methoxymethylphenol, 2,6-Di-nonyl-4-methylphenol.
   1.2. Alkylierte Hydrochinone, z.B. 2,6-Di-tert.butyl-4-methoxyphenol, 2,5-Di-tert.butyl-hydrochinon, 2,5-Di-tert.amyl-hydrochinon, 2,6-Diphenyl-4-octadecyioxyphenol.
   1.3. Hydroxylierte Thiodiphenylether, z.B. 2,2'-Thio-bis-(6-tert.-butyl-4-methylphenol), 2,2'-Thio-bis-(4-octytphenol), 4,4'-Thio-bis-(6-tert.butyl-3-methylphenol), 4,4'-Thio-bis-(6-tert.butyl-2-methylphenol).
   1.4. Alkyliden-Bisphenole, z.B. 2,2'-Methylen-bis-(6-tert.butyl-4-methylphenol), 2,2'-Methylen-bis-(6-tert.butyl-4-ethylphenol), 2,2'-Methylen-bis-[4-methyl-6-(α-methyl-cyclohexyl)-phenol], 2,2'-Methylen-bis-(4-methyl-6-cyclohexylphenol), 2,2'-Methylen-bis-(6-nonyl-4-methylphenol), 2,2'-Methylen-bis-(4,6-di-tert.butylphenol), 2,2'-Ethyliden-bis-(4,6-di-tert.butylphenol), 2,2'-Ethyliden-bis-(6-tert.butyl-4-isobutylphenol), 2,2'-Methylen-bis-[6-α-methylbenzyl)-4-nonylphenol], 2,2'-Methylen-bis-[6-(α,α-di-methylbenzyl)-4-nonylphenol], 4,4'-Methylen-bis-(2,6-di-tert.-butylphenol), 4,4'-Methylen-bis-(6-tert.butyl-2-methylphenol), 1,1-Bis-(5-tert.butyl-4-hydroxy-2-methyl-phenyl)-butan, 2,6-Bis-(3-tert.butyl-5-methyl-2-hydroxybenzyl)-4-methylphenol, 1,1,3-Tris-(5-tert.butyl-4-hydroxy-2-methylphenyl)-butan, 1,1-Bis-(5-tert.butyl-4-hydroxy-2-methyl-phenyl)-3-n-dodecylmercaptobutan, Ethylenglycol-bis- [3,3-bis-(3'-tert.-butyl-4'-hydroxyphenyl)-butyrat], Bis-(3-tert.butyl-4-hydroxy-5-methyl-phenyl)-dicyclopentadien, Bis-[2-(3'-tert.butyl-2'-hydroxy-5'-methyl-benzyl)-6-tert.butyl-4-methyl-phenyl]-terephthalat.
   1.5 Benzylverbindungen, z.B. 1,3,5-Tris-(3,5-di-tert.butyl-4-hydroxy-benzyl)-2,4,6-tri-methylbenzol, Bis-(3,5-di-tert.butyl-4-hydroxybenzyl)-sulfid, 3,5-Di-tert.butyl-4-hydroxybenzyl-mercaptoessigsäure-isooctylester, Bis-(4-tert.butyl-3-hydroxy-2,6-dimethyl-benzyl)dithiol-terephthalat, 1,3,5-Tris-(3,5-di-tert.butyl-4-hydroxybenzyl)-isocyanurat, 1,3,5-Tris-(4-tert.butyl-3-hydroxy-2,6-dimethylbenzyl)-isocyanurat, 3,5-Di-tert.butyl-4-hydroxybenzyl-phosphonsäure-dioctadecylester, Ca-Salz des 3,5-Di-tert.butyl-4-hydroxybenzyl-phosphonsäure-monoethylester, 1,3,5-Tris-(3,5-dicyclohexyl-4-hydroxybenzyl)isocyanurat.
   1.6. Acylaminophenole, z.B. 4-Hydroxy-laurinsäureanilid, 4-Hydroxystearinsäureanilid, 2,4-Bis-(octylmercapto)-6-(3,5-di-tert.butyl-4-hydroxyanilino )-s-triazin, N-(3,5-di-tert.-butyl-4-hydroxyphenyl)-carbaminsäureoctylester.
   1.7. Ester der β-(3,5-Di-tert.butyl-4-hydroxyphenyl)-propionsäure mit ein- oder mehrwenigen Alkoholen, wie z.B. mit Methanol, Octa- decanol, 1,6-Hexandiol, Neopentylglycol, Thiodiethylenglycol, Diethylenglycol, Triethylenglycol, Pentaerythrit, Tris(hydroxyethyl)-isocyanurat, N,N'-Bis-(hydroxyethyl)-oxalsäurediamid.
   1.8. Ester der β-(5-tert.Butyl-4-hydroxy-3-methylphenyl)-propionsäure mit ein- oder mehrwertigen Alkoholen, wie z.B. mit Methanol, Octadecanol, 1,6-Hexandiol, Neopentylglycol, Thiodiethylenglycol, Diethylenglycol, Triethylenglycol, Pentaerythrit, Tris-(hydroxy)ethyl-isocyanurat, N,N'-Bis-(hydroxyethyl)-oxalsäurediamid.
   1.9. Ester der β-(3,5-Dicyclohexyl-4-hydroxyphenyl)-propionsäure mit ein- oder mehrwenigen Alkoholen, wie z.B. mit Methanol, Octadecanol, 1,6-Hexandiol, Neopentylglycol, Thiodiethylenglycol, Diethylenglycol, Triethylenglycol, Pentaerythrit, Tris-(hydroxy)ethyl-isocyanurat, N,N'-Bis-(hydroxyethyl)-oxalsäurediamid.
   1.10. Amide der β-(3,5-Di-tert.butyl-4-hydroxyphenyl)-propionsäure, wie z.B. N,N'-Bis-(3,5-di-tert.butyl-4-hydroxyphenylpropionyl)-hexamethylendiamin, N,N'-Bis-(3,5-di-tert.-butyl-4-hydroxyphenylpropionyl)-trimethylendiamin, N,N'-Bis-(3,5-di-tert.butyl-4-hydroxyphenylpropionyl)-hydrazin.
2. UV-Absorber und Lichtschutzmittel
   2.1. 2-(2'-Hydroxyphenyl)-benztriazole, wie z.B. das 5'-Methyl-, 3',5'-Di-tert.butyl-, 5'-tert.Butyl-,5'-(1,1,3,3-Tetramethylbutyl)-, 5-Chlor-3',5' -di-tert.butyl-, 5-Chlor-3'-tert.-butyl-5'-methyl-, 3'-sec.Butyl-5'-tert.butyl, 4'-Octoxy-, 3',5'-Di-tert.amyl-, 3',5'-Bis-(α,α-dimethylbenzyl)-Derivat.
   2.2. 2-Hydroxybenzophenone, wie z.B. das 4-Hydroxy-, 4-Methoxy-, 4-Octoxy-, 4-Decyloxy-, 4-Dodecyloxy-, 4-Benzyloxy-, 4,2',4' -Trihydroxy-, 2' -Hydroxy-4,4' -dimerhoxy-Derivat.
   2.3. Ester von gegebenenfalls substituierten Benzoesäuren, wie z.B. 4-tert.Butyl-phenylsalicylat, Phenylsalicylat, Octylphenyl-salicylat, Dibenzoylresorcin, Bis-(4-tert.butyl-benzoyl)-resorcin, Benzoylresorcin, 3,5-Di-tert.butyl-4-hydroxybenzoesäure-2,4-di-tert.-butylphenylester, 3,5-Di-tert.butyl-4-hydroxybenzoesäurehexadecylester.
   2.4. Acrylate, wie z.B. α-Cyan-β,β-diphenylacrylsäure-ethylester bzw. -isooctylester, α-Carbomethoxy-zimtsäuremethylester, α-Cyano-β-methyl-p-methoxy-zimtsäuremethylester bzw. -butylester, α-Carbomethoxy-p-methoxy-zimtsäure-methylester, N-(β-Carbomethoxy-β-cyanovinyl)-2-methyl-indolin.
   2.5. Nickelverbindungen, wie z.B. Nickelkomplexe des 2,2'-Thio-bis-[4-(1,1,3,3-tetramethylbutyl)-phenols], wie der 1:1- oder der 1:2-Komplex, gegebenenfalls mit zusätzliehen Liganden, wie n-Butylamin, Triethanolamin oder N-Cyclohexyl-diethanolamin, Nickeldibutyldithiocarbamat, Nickelsalze von 4-Hydroxy-3,5-di-tert.butylbenzylphosphonsäure-monoalkylestern, wie vom Methyl- oder Ethylester, Nickelkomplexe von Ketoximen, wie von 2-Hydroxy-4-methyl-phenyl-undecylketoxim, Nickelkomplexe des 1-Phenyl-4-lauroyl-5-hydroxy-pyrazols, gegebenenfalls mit zusätzlichen Liganden.
   2.6. Sterisch gehinderte Amine, wie z.B. Bis-(2,2,6,6-tetramethyl-piperidyl)-sebacat, Bis-(1,2,2,6,6-pentamethylpiperidyl)-sebacat, n-Butyl-3,5-di-tert.butyl-4-hydroxybenzylmalonsäure-bis(1,2, 2,6,6-pentamethylpiperidyl)-ester, Kondensationsprodukt aus 1-Hydroxyethyl-2,2,6,6-tetramethyl-4-hydroxypiperidin und Bernsteinsäure, Kondensarionsprodukt aus N,N'-Bis-(2,2,6,6-Tetramethyl-4-piperidyl)-hexamethylendiamin und 4-tert.Octylamino-2,6-dichlor-1,3,5-s-triazin, Tris-(2,2,6,6-tetramethyl- 4-piperidyl)-nitrilotriacetat, Tetrakis-(2,2,6,6-tetramethyl-4-piperidyl)-1,2,3,4-butantetraoat, 1,1'-(1,2-Ethandiyl)-bis-(3,3,5,5-tetramethyl-piperazinon).
   2.7. Oxalsäurediamide, wie z.B. 4,4'-Di-octyloxy-oxanilid, 2,2'-Di-octyloxy-5,5'-di-tert.-butyl-oxanilid, 2,2'-Di-dodecyloxy-5,5'di-tert.butyl-oxanilid, 2-Ethoxy-2'-ethyl-oxanilid, N,N'-Bis-(3-dimethylaminopropyl)-oxalamid, 2-Ethoxy-5-tert.butyl-2'-ethyloxanilid und dessen Gemisch mit 2-Ethoxy-2'-ethyl-5,4'-di-tert.butyl-oxanilid, Gemische von o- und p-Methoxy- sowie von o- und p-Ethoxy-di-substituierten Oxaniliden.
   2.8. 2-(2-Hydroxyphenyl)-1,3,5-triazine, wie z.B. 2,4,6-Tris(2-hydroxy-4-octyloxyphenyl)-1,3,5-triazin, 2-(2-Hydroxy-4-octyloxyphenyl)-4,6-bis-(2,4-dimethylphenyl)-1,3,5-triazin, 2-(2,4-Dihydroxyphenyl)-4,6-bis(2,4-dimethylphenyl)-1,3,5-triazin, 2,4-Bis-(2-hydroxy-4-propyloxyphenyl)-6-(2,4-dimethylphenyl)-1,3,5-triazin, 2-(2-Hydroxy-4-octyloxyphenyl)-4,6-bis(4-methylphenyl)-1,3,5-triazin, 2-(2-Hydroxy-4-dodecyloxyphenyl)-4,6-bis(2,4-dimethylphenyl)-1,3,5-triazin.
3. Metalldesaktivatoren, wie z.B. N,N'-Diphenyloxalsäurediamid, N-Salicylal-N'-salicyloylhydrazin, N,N'-Bis-(salicyloyl)-hydrazin, N,N'-Bis-(3,5-di-terubutyl-4-hydroxyphenylpropionyl)-hydrazi n, 3-Salicyloylamino-1,2,4-triazol, Bis-(benzyliden)-oxalsäuredihydrazid.
4. Phosphite und Phosphonite, wie z.B. Triphenylphosphit, Diphenylalkylphosphite, Phenyldialkylphosphite, Tris-(nonylphenyl)-phosphit, Trilaurylphosphit, Trioctadecylphosphit, Distearyl-pentaerythritdiphosphit, Tris-(2,4-di-tert.butylphenyl)-phosphit, Diisodecylpentaerythrit-diphosphit, Bis-(2,4-di-tert.butylphenyl)-pentaerythritdiphosphit, Tristearyl-sorbit-triphosphit, Tetrakis-(2,4-di-tert.butylphenyl)-4,4'-biphenylen-diphosphonit, 3,9-Bis-(2,4-di-tert.butylphenoxy)-2,4,8,10-tetraoxa-3,9-diphosphaspiro[5.5]undecan.
5. Peroxidzerstörende Verbindungen, wie z.B. Ester der β-Thio-dipropionsäure, beispielsweise der Lauryl-, Stearyl-, Myristyl- oder Tridecylester, Mercaptobenzimidazol, das Zinksalz des 2-Mercaptobenzimidazols, Zink-dibutyl-dithiocarbamat, Dioctadecyldisulfid, Pentaerythrit-tetrakis-(β-dodecylmercapto)-propionat.
6. Polyamidstabilisatoren, wie z.B. Kupfersalze in Kombination mit Jodiden und/oder Phosphorverbindungen und Salze des zweiwertigen Mangans.
7. Basische Co-Stabilisatoren, wie z.B. Melamin, Polyvinylpyrrolidon, Dicyandiamid, Triallylcyanurat, Harnstoff-Derivate, Hydrazin-Derivate, Amine, Polyamide, Potyurethane, Alkali- und Erdalkalisalze höherer Fettsäuren, beispielsweise Ca-Stearat, Zn-Stearat, Mg-Stearat, Na-Ricinoleat, K-Palmitat, Antimonbrenzcatechinat oder Zinnbrenzcatechinat.
8. Nukleierungsmittel, wie z.B. 4-tert.Butylbenzoesäure, Adipinsäure, Diphenylessigsäure.
9. Füllstoffe und Verstärkungsmittel, wie z.B. Calciumcarbonat, Silikate, Glasfasern, Asbest, Talk, Kaolin, Glimmer, Bariumsulfat, Metalloxide und -hydroxide, Russ, Graphit.
10. Sonstige Zusätze, wie z.B. Weichmacher, Gleitmittel, Emulgatoren, Pigmente, Optische Aufhellen, Flammschutzmittel, Antistatika, Treibmittel.

Ausserdem können sonstige Materialien, wie sie in der Technologie der Kunststoffe und Lacke üblich sind, zugesetzt werden. Beispiele hierfür sind Füllstoffe und Verstärkungsmittel, Pigmente, Farbstoffe, Weichmacher, Lösungsmittel, Gleitmittel, Verlaufhilfsmittel, optische Aufheller, Nukleierungsmittel, Antistatika, Flammschutzmittel oder Katalysatoren (z.B. Säuren, Metallseifen oder -salze, Amine).

Die Art der zugesetzten weiteren Stabilisatoren wird selbstverständlich von der Art des zu stabilisierenden Substrates und dessen Verwendungszweck bestimmt.

Die so stabilisierten Polymeren können in verschiedener Form angewendet werden, z.B. als Folien, Fasern, Bändchen, Formteile, Profile, Latex, Dispersionen, Lacke oder Kitte.

Die erfindungsgemässen Verbindungen können als Stabilisatoren, insbesondere als Lichtschutzmittel, auch für praktisch alle in der Reproduktionstechnik bekannten Materialien verwendet werden, wie dies in Research Disclosure 90/31429 [No. 314, S, 474-480 (1990)] beschrieben ist.

Die vorliegende Erfindung betrifft somit auch Aufzeichnungsmaterialien, wie in der oben erwähnten Literaturstelle beschrieben, insbesondere Photomaterialien, vor allem farbphotographische Materialien, die in mindestens einer Schicht mindestens eine Verbindung gemäss vorliegender Erfindung enthalten.

Die nachfolgenden Beispiele illustrieren die Erfindung näher, ohne sie auf die Beispiele beschränken zu wollen. Darin sowie in der übrigen Beschreibung und in den Patentansprüchen bedeuten Teile und Prozente Gewichtsteile und Gewichtsprozente, sofern nichts anderes angegeben ist. EA bedeutet Elementaranalyse.

### Beispiel 1:

14,5 g (0,03 Mol) 2-(2-Hydroxy-4-ethoxycarbonylmethoxy-phenyl)-4,6-bis-(2,4-dimethylphenyl)-1,3,5-triazin (hergestellt wie in CH-A 484 695, Beispiel 44 beschrieben) werden in einem 100 ml Sulfierkolben vorgelegt. Dazu gibt man 4,65 g (0,03 Mol) 4-Hydroxy-2,2,6,6-tetramethylpiperidin und 0,1 g Dibutylzinnoxid als Katalysator. Man erhitzt das Gemisch auf 150°C Innentemperatur und lässt langsam aus einem Tropftrichter, unter Rühren, eine Lösung von 0,4 g 4-Hydroxy-2,2,6,6-tetramethylpiperidin in 100 ml Toluol so zutropfen, dass ein Toluol/Ethanol-Gemisch durch die Umesterung laufend abdestilliert wird. Nach ca. 6 Std. Reaktionszeit zeigt das Dünnschichtchromatogramm (Laufmittel Toluol/20 % Ethanol) kein Ausgangsprodukt (Hydroxyphenyltriazin) mehr. Danach versetzt man die gelbliche Schmelze mit 250 ml Toluol, kühlt die Lösung ab, wäscht die organische Phase 2 mal mit je 50 ml warmem Wasser und dampft danach die Lösung am Rotationsverdampfer ein. Der Rückstand wird aus Acetonitril umkristallisiert. Man erhält das Produkt (Verbindung 1) als beiges Kristallisat mit Smp.: 118-120°C (siehe Tabelle I).

Verwendet man anstelle des 4-Hydroxy-2,2,6,6-tetramethylpiperidins andere in 1-Stellung substituierte Piperidine und verfährt im übrigen genau gleich wie in Beispiel 1 beschrieben, so erhält man die Verbindungen 2-7 in Tabelle 1. Anstelle des Bis-Xylyl-derivates des Hydroxyphenyltriazins kann auch das Bis-Toluylderivat verwendet werden (Verbindungen 8-11 in Tabelle I, Beispiel 2).

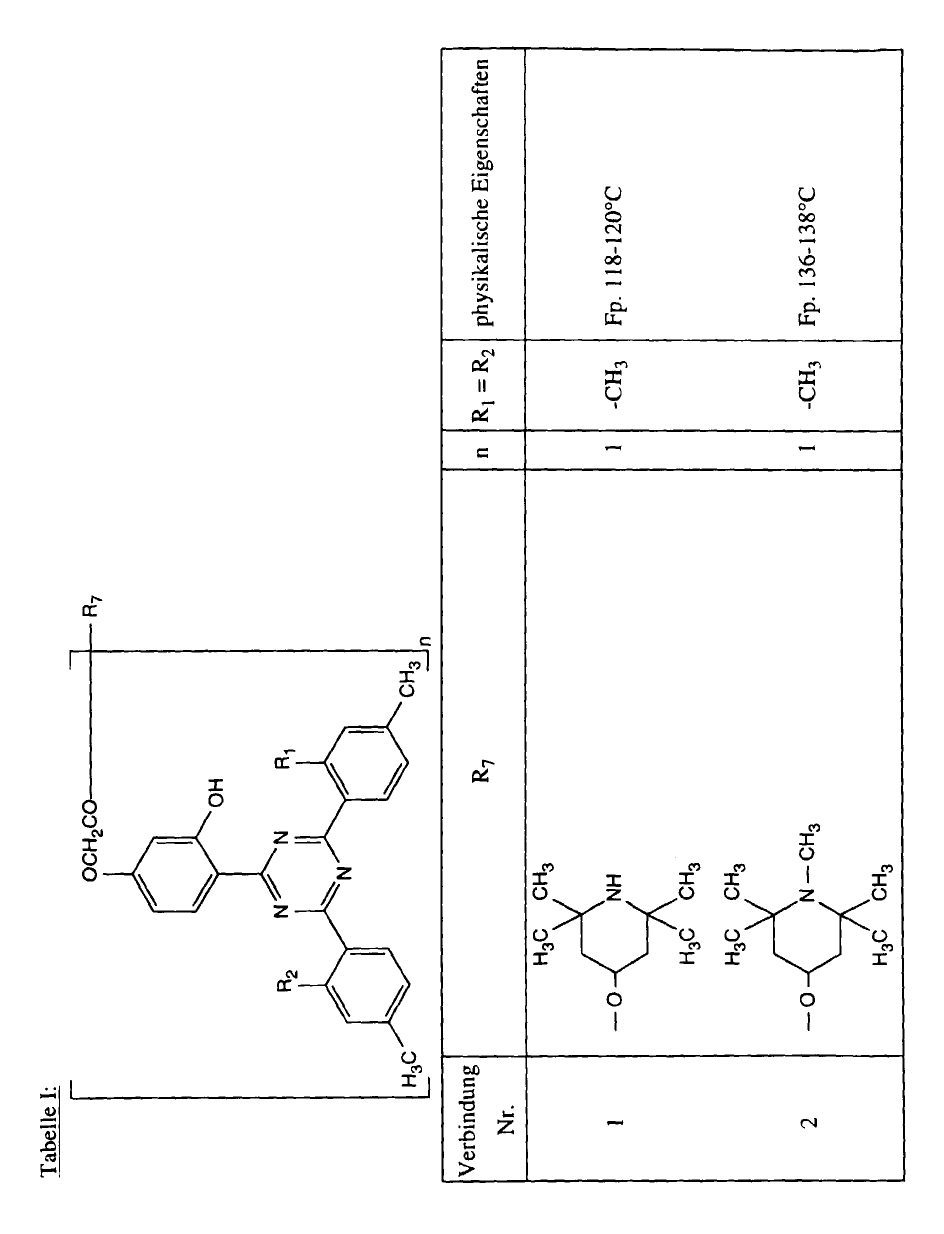

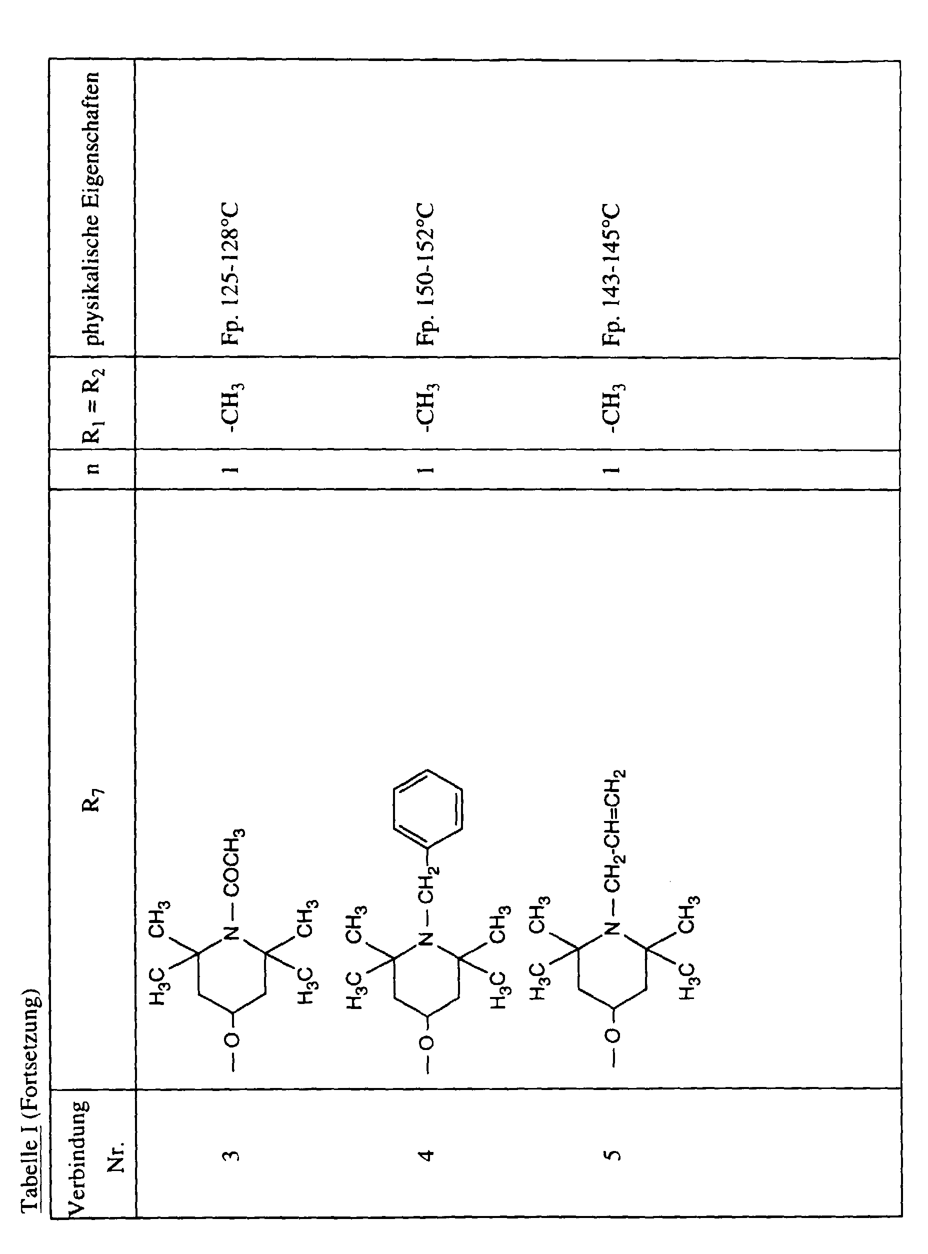

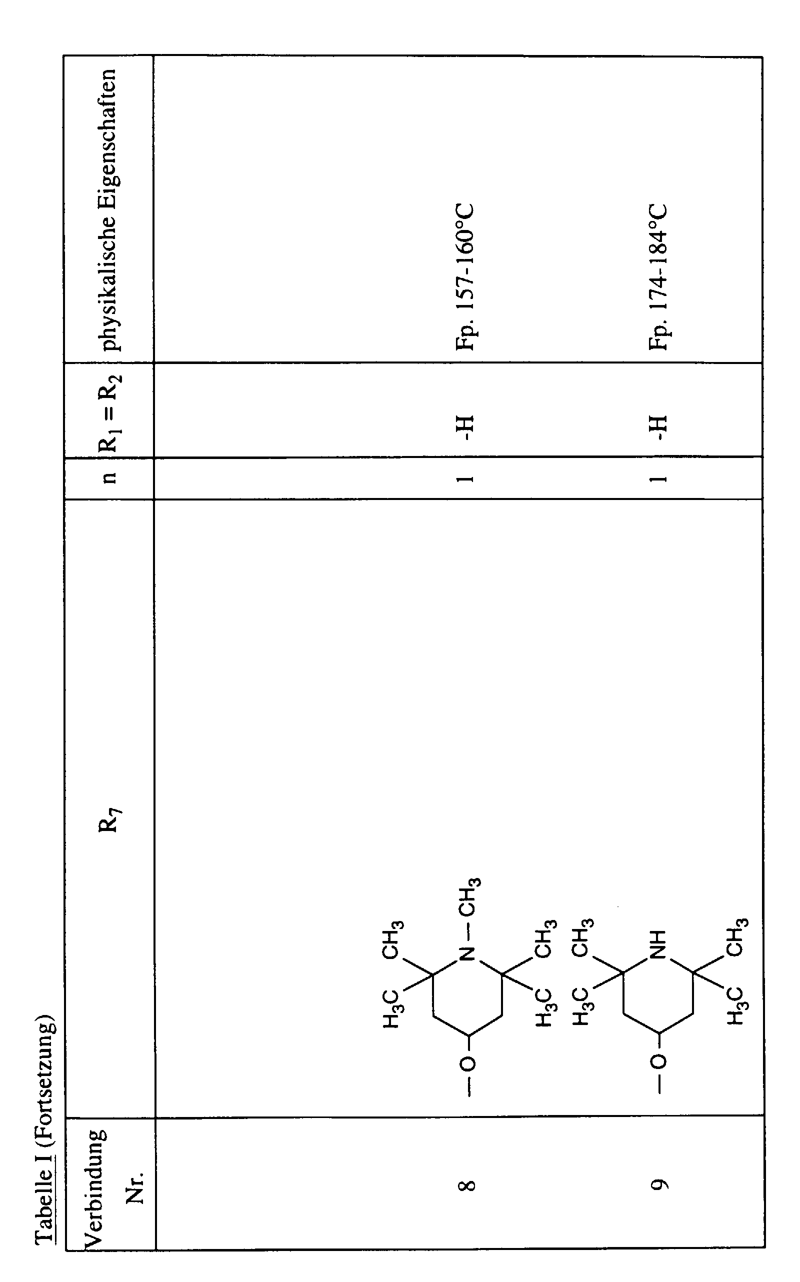

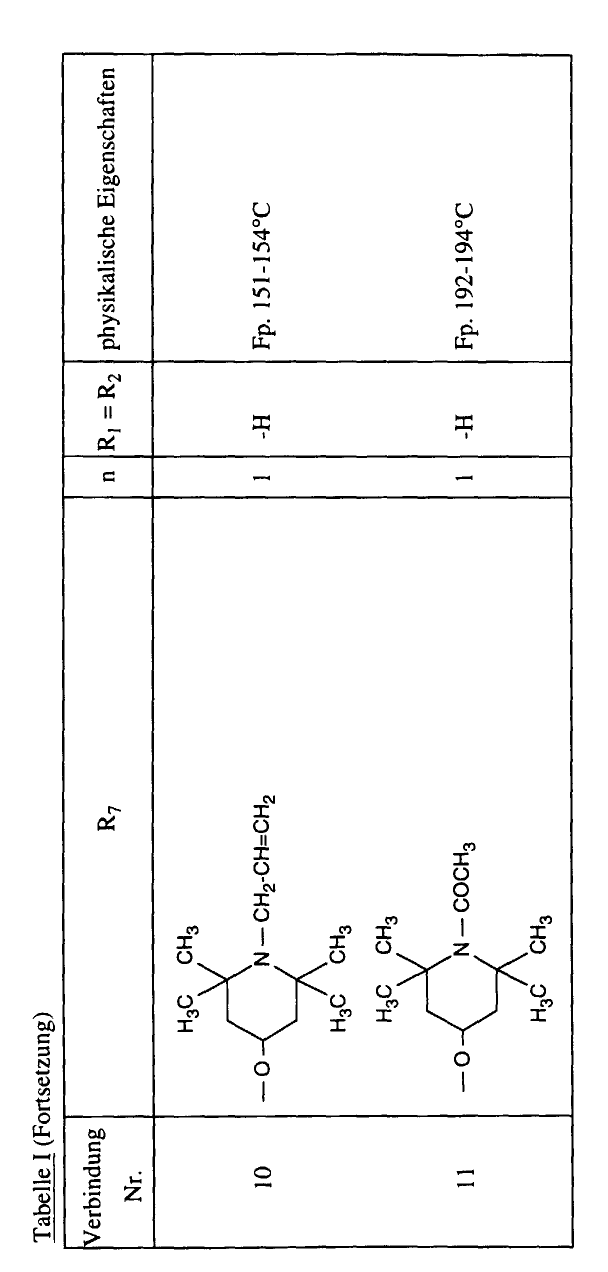

Beispiel 2:

In einem 200 ml Sulfierkolben wird eine Suspension von 9,1 g (0,02 Mol) 2-(2-Hydroxy-4-ethoxycarbonylmethoxy-phenyl)-4,6-bis-(4-methylphenyl)-1,3,5-triazin, 4,1 g (0,024 Mol) 4-Hydroxy-1,2,2,6,6-pentamethylpiperidin und 0,5 g (2 mMol) Dibutylzinnoxid in 90 ml reinem Xylol (Isomerengemisch) zum Rückfluss erhitzt. Der entstandene Ethanol wird mit einem Dean-Stark Wasserabscheider entfernt. Nach 21 Stunden filtriert man das Reaktionsgemisch und verdampft das Lösungsmittel. Das beige feste Rohprodukt wird aus 500 ml Benzin (Siedegrenze 110-140°C) umkristallisiert und getrocknet. Man erhält 9,9 g (85 % d. Th.) der Verbindung Nr. 8 als leicht gelbes Pulver; Smp. 157-160°C.

In analoger Weise werden durch Umsetzung mit weiteren 4-Hydroxytetraalkylpiperidinen die Verbindungen 9-11 erhalten.

### Beispiel 4:

In einem 200 ml Sulfierkolben werden nacheinander folgende Verbindungen vorgelegt: 39,7 g (0,1 Mol) 2-(2,4-Dihydroxyphenyl)-4,6-bis(2,4-dimethylphenyl)-1,3,5-triazin, 29,3 g (0,105 Mol) 4-(2-Epoxypropyloxy)-N-(propen-2-yl)-2,2,6,6-tetramethylpiperidin, 50 ml Xylol (Isomerengemisch) und 0,33 g (0,004 Mol) N-Methylimidazol. Das Gemisch wird 2 Stunden auf 145°C erhitzt. Dabei entsteht eine dunkelgelbe Reaktionslösung. Nach 2 Stunden Reaktionszeit zeigt das Dünnschichtchromatogramm kein Ausgangsprodukt mehr. Das Reaktionsgemisch wird abgekühlt, über 150 g Kieselgel filtriert, mit Xylol nachgespült, und der Katalysator wird mit dreimal je 100 ml Wasser ausgeschüttelt. Die organische Phase wird über Na₂SO₄ getrocknet und eingedampft. Es entsteht ein gelbes glasiges Harz (Verbindung Nr. 13) (44,5 g = 68,4 % d.Th.).

| | | | | |
|---|---|---|---|---|
| EA: | berechnet | C 73,81 % | H 7,74 % | N 8,60 % |
| | gefunden | C 73,65 % | H 7,66 % | N 8,50 %. |

In analoger Weise werden durch Umsetzung mit weiteren 4-Glycidyloxytetramethylpiperidinen die in Tabelle II angegebenen Verbindungen Nr. 14-16 erhalten.

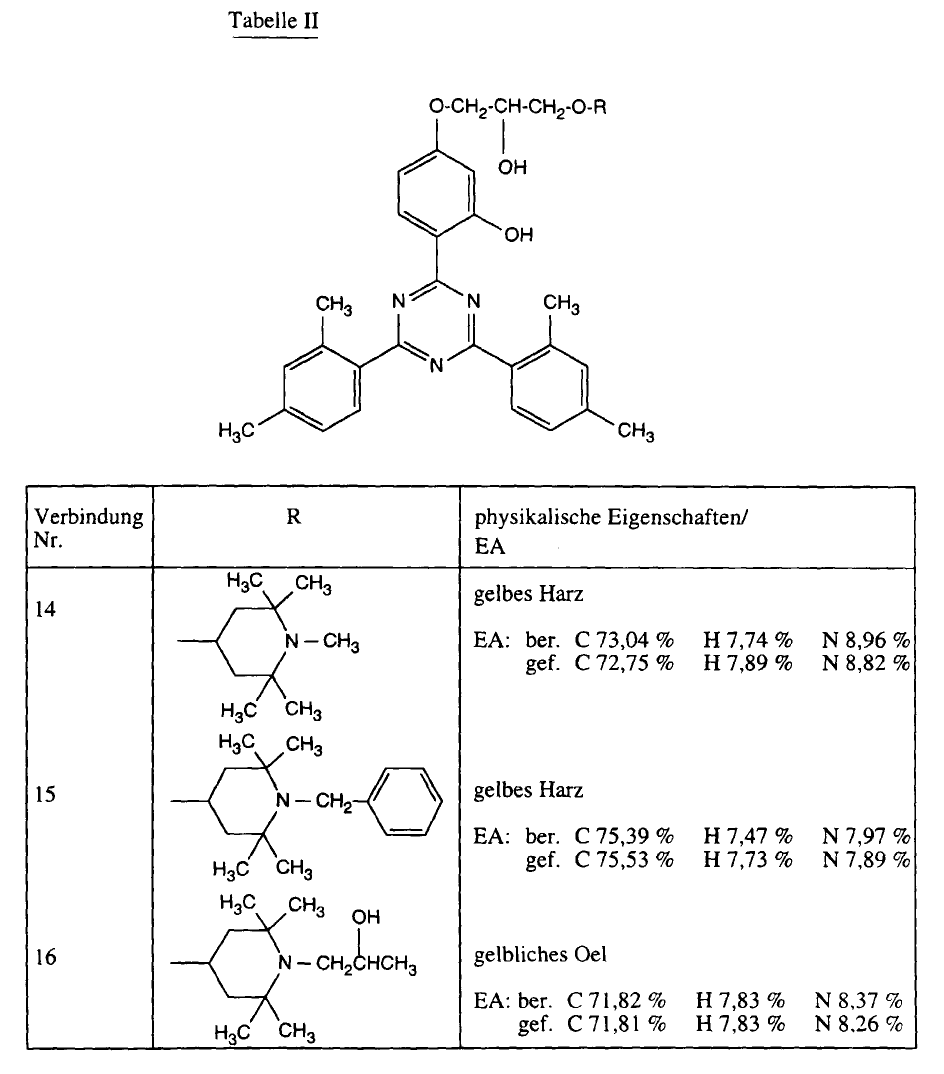

### Beispiel 6: Prüfung erfindungsgemässer Verbindungen in einem Zweikomponenten-Polyacrylat-Lack

Ein hydroxylgruppenhaltiger Acrylat-Lack wird durch Mischen der folgenden Komponenten hergestellt:
75 Teile einer 60%igen Lösung eines hydroxylgruppenhaltigen Acrylatharzes in Xylol (Macrynal ®SM 510N, Hoechst AG)
15 Teile Butylglykolacetat
6,1 Teile eines aromatischen Lösungsmittelgemisches (Solvesso ® 100, Esso AG)
3,6 Teile Methyl-isobutylketon
0,1 Teile Zink-octoat (als 8%ige Lösung in Toluol)
0,2 Teile eines Verlaufhilfsmittels (Byk® 300, Byk-Chemie).

Dazu werden 1,5 Teile der unten angegebenen Stabilisatoren (gelöst in 5-10 Teilen Xylol) zugegeben. Dies entspricht 2 % Stabilisator, bezogen auf festes Bindemittel (Acrylat + Isocyanat). Die erhaltenen Formulierungen werden mit 30 Teilen eines trimerisierten Diisocyanates (Desmodur® N 75, Bayer AG) vermischt. Der Klarlack wird mit Xylol auf Spritzfähigkeit eingestellt. Proben des Lackes werden auf Aluminiumbleche, die mit einem Coil-coat, einem Automobilfüller auf Polyesterbasis und einem Silbermetallic-Basislack auf Polyester/Celluloseacetobutyrat/Melamin-Basis beschichtet sind, aufgetragen und 45 Minuten bei 80°C eingebrannt. Es resultiert eine Schichtdicke von 45-50 µm.

Die Probenbleche werden in einem Xenon-Weatherometer (Atlas Corp., CAM 159, KFA-Methode) bewittert. Es werden der 20° Glanz nach DIN 67530 vor der Bewitterung und nach 800, 1600 und 2000 Stunden sowie die Zeit (in Std.) bis zur Rissbildung und das Aussehen des Lackes gemäss TNO-Rissbildungsskala, Beschreibung 353/D, bestimmt.

| Stabilisator | 20° Glanz nach .... Stunden | | | | Rissbildung nach ... Stunden/Aussehen |
|---|---|---|---|---|---|
| | 0 | 800 | 1600 | 2000 | |
| ohne Stabilisator | 87 | 87 | 2 | - | 1600, matt |
| Stabilisator Nr. 1 | 87 | 88 | 86 | 80 | 2800, E4b |
| Stabilisator Nr. 2 | 89 | 89 | 89 | 89 | 2400, E4b |

Aus den obigen Daten sind die mit den erfindungsgemässen Verbindungen erzielbare gute Glanzhaltung und hohe Rissbeständigkeit ersichtlich.

## Patentansprüche

1. Verbindung der Formel Ia 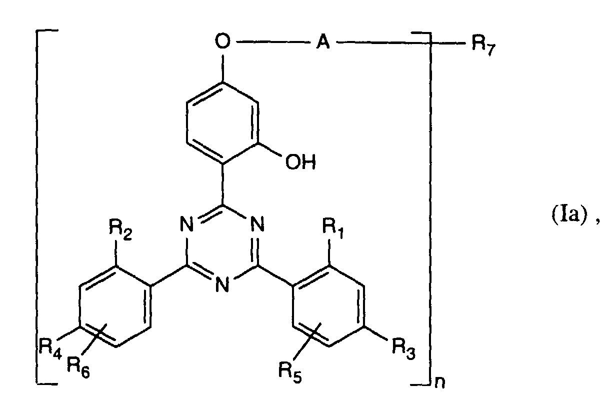
n 1 ist,
R₁ und R₂ unabhängig voneinander Wasserstoff, -OH, C₁-C₁₂-Alkyl oder Halogen,
R₃ und R₄ unabhängig voneinander Wasserstoff, -OH, C₁-C₁₂-Alkyl, C₁-C₁₈-Alkoxy, Halogen oder einen Rest -O-A-R₇ darstellen,
R₅ und R₆ unabhängig voneinander Wasserstoff, C₁-C₁₂-Alkyl, Halogen oder einen Rest -O-A-R₇ bedeuten,
A eine direkte Bindung, -(CH₂)ₘCO- mit m = Null oder 1 bis 4, -CH₂CH(R')O- oder -CH₂CH(OH)CH₂- ist,
R₇ ein Rest der Formeln V bis VIIb oder Xa oder Xb ist: 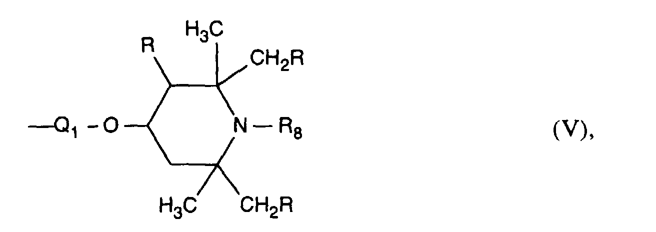 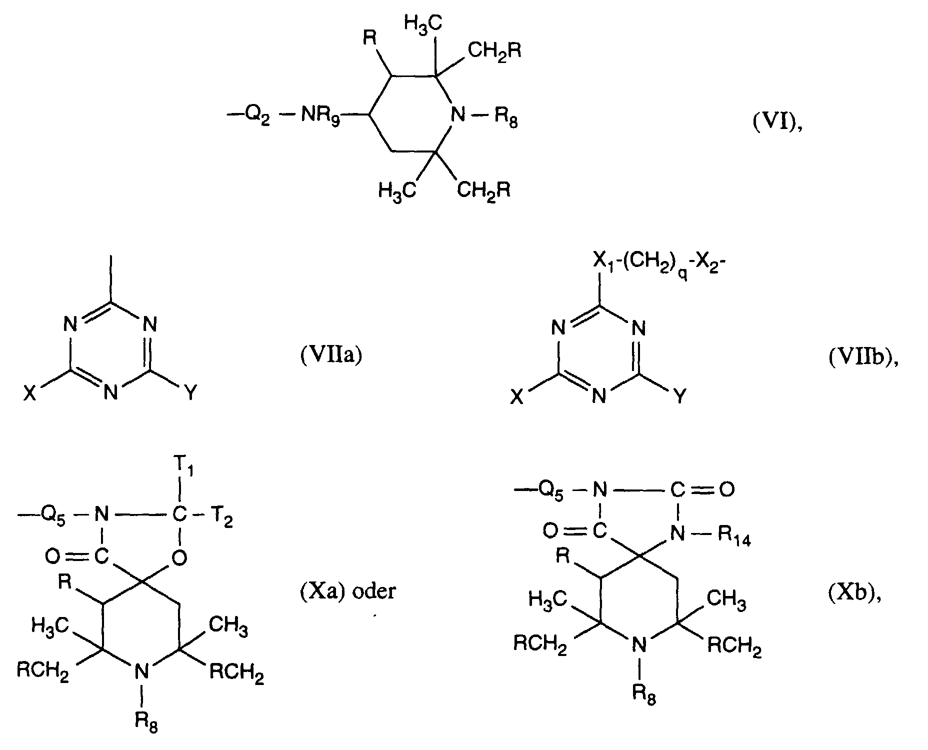 wobei Reste der Formeln VIIa nur in Verbindungen der Formel Ia mit A = direkte Bindung oder -CH₂CH(R')O- und Reste der Formeln VIIb in Verbindungen der Formel Ia vorliegen, worin A ungleich direkte Bindung oder -CH₂CH(R')O- ist, und worin
R Methyl und insbesondere Wasserstoff ist,
R₈ Wasserstoff, Oxyl, C₁-C₁₂-Alkyl, C₁-C₁₂-Alkoxy, C₃-C₈-Alkenyl, C₃-C₈-Alkenyloxy, C₃-C₈-Alkinyl, C₅-C₆-Cycloalkyl, C₅-C₆-Cycloalkyloxy, Phenyl-C₁-C₄-alkyl, C₁-C₈-Alkanoyl, C₃-C₅-Alkenoyl, -CH₂CH(OH)R' oder Phenoxy bedeutet,
R' Wasserstoff, Methyl oder Phenyl ist,
R₉ Wasserstoff, C₁ -C₁₈-Alkyl, Allyl, C₅-C₆-Cycloalkyl, Phenyl-C₁-C₄-alkyl, durch ein oder mehrere -O-unterbrochenes C₃-C₁₈-Alkyl oder eine Gruppe der Formel IIIa 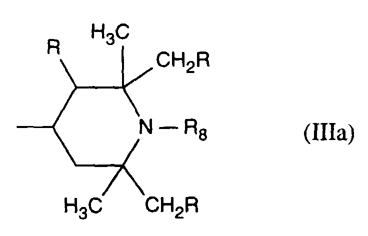 darstellt,
Q₁ die direkte Bindung, -O-C₂-C₁₂-Alkylen oder -NR₉-C₂-C₁₂-Alkylen ist, wenn A die angegebene Bedeutung haben, A aber ungleich direkte Bindung oder -CH₂CH(R')O- ist, oder in Verbindungen der Formel Ia Q₁ C₂-C₁₂-Alkylen ist, wenn A die direkte Bindung oder -CH₂CH(R')O- darstellt, oder -OCO-C₂-C₁₂-Alkylen darstellt, wenn A in Verbindungen der Formel Ia -CH₂CH(OH)CH₂- ist,
Q₂ die direkte Bindung oder -NR₉-C₂-C₁₂-Alkylen ist, wenn A ungleich direkte Bindung oder -CH₂CH(R')O- ist, oder in Verbindungen der Formel Ia Q₂ -CO-C₂-C₁₂-Alkylen darstellt, wenn A die direkte Bindung oder -CH₂CH(R')O- ist, oder -OCO-C₁-C₁₂-Alkylen oder -OCO-C₁-C₁₂-Alkylen-CO- ist, wenn A in Formel Ia -CH₂CH(OH)CH₂- ist, q 2-12, besonders 2-4 ist,
X₁ und X₂ unabhängig voneinander -O- oder -R₉N- darstellen, 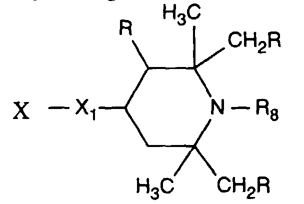 ist und Y dieselbe Bedeutung wie X hat oder C₁-C₁₈-Alkoxy oder -NR₉R₁₀ bedeutet,
R₁₀ die gleiche Bedeutung wie R₉ haben kann oder zusammen mit R₉ und dem Bindungs-N-Atom einen 5- oder 6-gliedrigen heterocyclischen Ring bildet,
Q₅ -OCH₂CH₂- oder -OCH₂CH(OH)CH₂- ist, wenn A oder B die angegebene Bedeutung haben, A aber ungleich direkte Bindung oder -CH₂CH(R')O- ist, oder in Verbindungen der Formel Ia Q₅ -CO(CH₂)₂- oder -CO(CH₂)₃- ist, wenn A die direkte Bindung oder -CH₂CH(R')O- darstellt, Q₅ die direkte Bindung bedeutet, wenn A -CH₂CH(OH)CH₂- ist, oder Q₅ -OCO(CH₂)ₛ- mit s = 1-4 ist, wenn A in Formel Ia -CH₂CH(OH)CH₂- ist,
T₁ und T₂ unabhängig voneinander Wasserstoff, C₁-C₁₈-Alkyl, Phenyl-C₁-C₄-alkyl oder unsubstituiertes oder durch Halogen oder C₁-C₄-Alkyl substituiertes Phenyl oder Naphthyl bedeuten, oder T₁ und T₂ zusammen mit dem sie verbindenden C-Atom einen C₅-C₁₂-Cycloalkanring bilden,
R₁₄ Wasserstoff, C₁-C₁₂-Alkyl, Allyl, Benzyl oder C₂-C₆-Alkoxyalkyl ist.

2. Verbindung nach Anspruch 1, worin R Wasserstoff, R' Wasserstoff oder Methyl und m und s je die Zahl 1 sind und R₁ und R₂, R₃ und R₄ sowie R₅ und R₆ je paarweise dieselbe Bedeutung haben.

3. Verbindung nach Anspruch 1, worin R₁ und R₂ sowie R₃ und R₄ je paarweise Wasserstoff oder C₁-C₄-Alkyl bedeuten und R₅ und R₆ je Wasserstoff sind.

4. Verbindung nach Anspruch 1, worin in Formel Ia R₁ und R₂ je Wasserstoff, -OH oder Methyl, insbesondere Wasserstoff oder Methyl bedeuten, R₃ und R₄ je Wasserstoff, -OH oder Methyl, insbesondere Wasserstoff und besonders Methyl darstellen, und R₅ und R₆ je Wasserstoff sind.

5. Verbindung nach Anspruch 1, worin R₈ Wasserstoff, Oxyl, C₁-C₁₂-Alkyl, C₃-C₈-Alkenyl, C₃-C₈-Alkinyl, Phenyl-C₁-C₄-alkyl, C₁-C₈-Alkanoyl, C₃-C₈-Alkenoyl oder -CH₂CH(OH)R', insbesondere Wasserstoff, Oxyl, C₁-C₈-Alkyl, Allyl, Benzyl, Acetyl oder Acryloyl ist.

6. Verbindung nach Anspruch 1 oder 5, worin R₉ und R₁₀ Wasserstoff, C₁-C₈-Alkyl, Allyl, Cyclohexyl, Benzyl oder eine Gruppe der Formel IIIa, worin R Wasserstoff ist und R₈ die in Anspruch 5 angegebene Bedeutung hat, und insbesondere C₁-C₄-Alkyl oder Wasserstoff ist.

7. Verbindung der Formel Ia nach Anspruch 1, worin A -CH₂CO- ist, R₁ und R₂ sowie R₃ und R₄ je paarweise Wasserstoff oder C₁-C₄-Alkyl darstellen, R₅ und R₆ je Wasserstoff sind, R₇ einen Rest der Formel V darstellt, Q₁ die direkte Bindung ist, R₈ Wasserstoff, C₁-C₄-Alkyl, Benzyl, Allyl, Acetyl oder Acryloyl bedeutet, und R' Wasserstoff, Methyl oder Phenyl darstellt.

8. Verbindung der Formel Ia nach Anspruch 1, worin A -CH₂CO- ist, R₁ und R₂ je Wasserstoff und insbesondere je Methyl darstellen, R₃ und R₄ je Methyl und R₅ und R₆ je Wasserstoff sind und R₇ einen Rest der Formel V bedeutet, worin Q₁ die direkte Bindung, R₈ Wasserstoff, Methyl, Benzyl, Allyl oder Acetyl, und R' Wasserstoff oder Methyl darstellen.

9. Verbindung der Formel Ia nach Anspruch 1, worin A -CH₂CH(OH)CH₂- ist, R₁ und R₂ sowie R₃ und R₄ je paarweise Wasserstoff oder C₁-C₄-Alkyl darstellen, R₅ und R₆ je Wasserstoff sind, R₇ einen Rest der Formel V darstellt, Q₁ die direkte Bindung ist, R₈ Wasserstoff, C₁-C₄-Alkyl, Benzyl, Allyl, Acetyl oder -CH₂CH(OH)R' ist.

10. Verbindung der Formel Ia nach Anspruch 1, worin A -CH₂CH(OH)CH₂- ist, R₁ und R₂ je Wasserstoff und insbesondere je Methyl darstellen. R₃ und R₄ je Methyl und R₅ und R₆ je Wasserstoff sind und R₇ einen Rest der Formel V darstellt, Q₁ die direkte Bindung ist, R₈ Methyl, Benzyl, Allyl oder -CH₂CH(OH)CH₃ ist.

11. Organisches Material, enthaltend als Stabilisator mindestens eine Verbindung nach Anspruch 1.

12. Organisches Material nach Anspruch 11, worin das organische Material ein organisches Polymer ist.

13. Organisches Material nach Anspruch 11, worin das organische Material ein Anstrichstoff ist.

14. Organisches Material nach Anspruch 11, worin das organische Material ein Aufzeichnungsmaterial ist.

15. Verwendung von Verbindungen nach Anspruch 1 als Stabilisatoren für organische Materialien gegen Schädigung durch Licht, Sauerstoff und/oder Hitze.

## Claims

1. A compound of the formula Ia 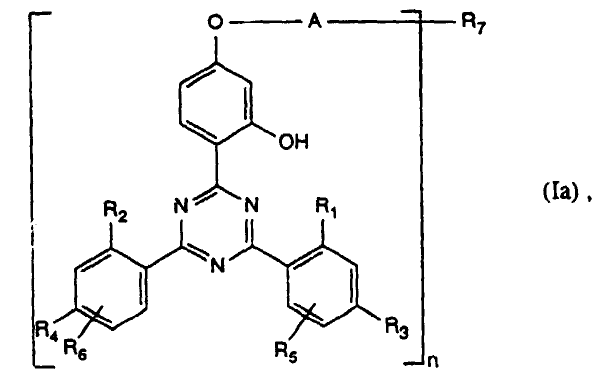 in which
n is 1,
R₁ and R₂ independently of one another are hydrogen, -OH, C₁-C₁₂alkyl or halogen,
R₃ and R₄ independently of one another are hydrogen, -OH, C₁-C₁₂alkyl, C₁-C₁₈alkoxy, halogen or a radical -O-A-R₇,
R₅ and R₆ independently of one another are hydrogen, C₁-C₁₂alkyl, halogen or a radical -O-A-R₇,
A is a direct bond, -(CH₂)ₘCO- where m = zero or 1 to 4, -CH₂-CH(R')O- or -CH₂CH(OH)CH₂-,
R₇ is a radical of the formulae V to VIIb or Xa or Xb: 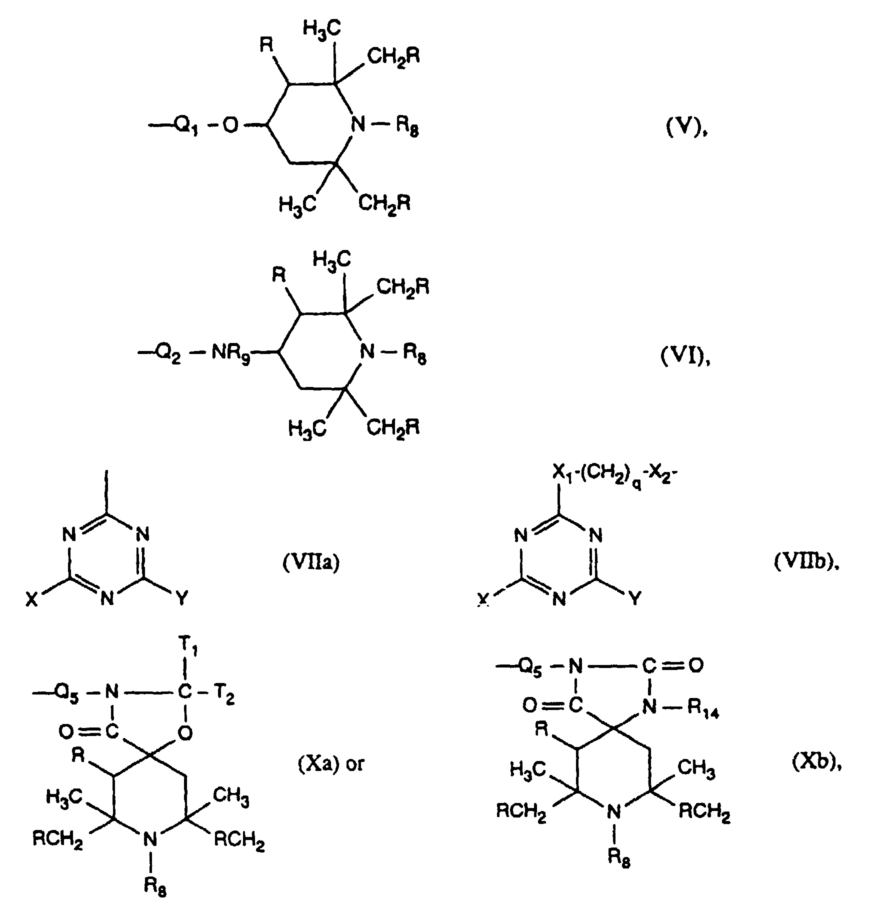 where radicals of the formulae VIIa are present only in compounds of the formula Ia where A = a direct bond or -CH₂CH(R')O- and radicals of the formulae VIIb are present in compounds of the formula Ia in which A is not equal to a direct bond or -CH₂CH(R')O-, and in which
R is methyl and in particular hydrogen,
R₈ is hydrogen, oxy, C₁-C₁₂alkyl, C₁-C₁₂alkoxy, C₃-C₈alkenyl, C₃-C₈alkenyloxy, C₃-C₈alkynyl, C₅-C₆cycloalkyl, C₅-C₆cycloalkylkoxy, phenyl-C₁-C₄alkyl, C₁-C₈alkanoyl, C₃-C₅alkenoyl, -CH₂CH(OH)R' or phenoxy,
R' is hydrogen, methyl or phenyl,
R₉ is hydrogen, C₁-C₁₈alkyl, allyl, C₅-C₆cycloalkyl, phenyl-C₁-C₄alkyl, C₃-C₁₈alkyl interrupted by one or more -O- or a group of the formula IIIa 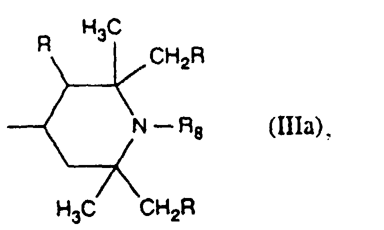
Q₁ is a direct bond, -O-C₂-C₁₂alkylene or -NR₉-C₂-C₁₂alkylene if A has the meaning indicated, but A is not equal to a direct bond or -CH₂CH(R')O-, or in compounds of the formula Ia Q₁ is C₂-C₁₂alkylene if A is a direct bond or -CH₂CH(R')O-, or -OCO-C₂-C₁₂alkylene if A in compounds of the formula Ia is -CH₂CH(OH)CH₂-,
Q₂ is a direct bond or -NR₉-C₂-C₁₂alkylene if A is not equal to a direct bond or -CH₂CH(R')O-, or in compounds of the formula Ia Q₂ is -CO-C₂-C₁₂alkylene if A is a direct bond or -CH₂CH(R')O-, or is -OCO-C₁-C₁₂alkylene or -OCO-C₁-C₁₂alkylene-CO- if A in formula Ia is -CH₂CH(OH)CH₂-,
q is 2-12, especially 2-4,
X₁ and X₂ independently of one another are -O- or -R₉N-, 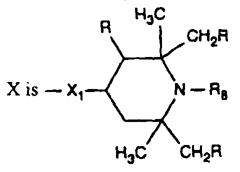 and Y has the same meaning as X or is C₁-C₁₈alkoxy or -NR₉-R₁₀,
R₁₀ can have the same meaning as R₉ or, together with R₉ and the bonding N atoms, forms a 5- or 6-membered heterocyclic ring,
Q₅ is -OCH₂CH₂- or -OCH₂CH(OH)CH₂- if A or B has the meaning indicated, but A is not equal to a direct bond or -CH₂CH(R')O-, or in compounds of the formula Ia Q5 is -CO(CH₂)₂- or -CO(CH₂)₃- if A is a direct bond or -CH₂CH(R')O-, Q₅ is a direct bond if A is -CH₂CH(OH)CH₂-, or Q₅ is -OCO(CH₂)ₛ- where s = 1-4 if A in formula Ia is -CH₂CH(OH)CH₂-,
T₁ and T₂ independently of one another are hydrogen, C₁-C₁₈alkyl, phenyl-C₁-C₄alkyl or phenyl or naphthyl which are unsubstituted or substituted by halogen or C₁-C₄alkyl, or T₁ and T₂, together with the C atom bonding them, form a C₅-C₁₂cycloalkane ring,
R₁₄ is hydrogen, C₁-C₁₂alkyl, allyl, benzyl or C₂-C₆alkoxyalkyl.

2. A compound according to claim 1, in which R is hydrogen, R' is hydrogen or methyl and m and s are each the number 1 and R₁ and R₂, R₃ and R₄ and also R₅ and R₆ in pairs each have the same meaning.

3. A compound according to claim 1, in which R₁ and R₂ and also R₃ and R₄ in pairs are each hydrogen or C₁-C₄alkyl and R₅ and R₆ are each hydrogen.

4. A compound according to claim 1, in which in formula Ia R₁ and R₂ are each hydrogen, -OH or methyl, in particular hydrogen or methyl, R₃ and R₄ are each hydrogen, -OH or methyl, in particular hydrogen and especially methyl, and R₅ and R₆ are each hydrogen.

5. A compound according to claim 1, in which R₈ is hydrogen, oxy, C₁-C₁₂alkyl, C₃-C₈alkenyl, C₃-C₈alkynyl, phenyl-C₁-C₄alkyl, C₁-C₈alkanoyl, C₃-C₈alkenoyl or -CH₂CH(OH)R', in particular hydrogen, oxy, C₁-C₈alkyl, allyl, benzyl, acetyl or acryloyl.

6. A compound according to claim 1 or 5, in which R₉ and R₁₀ are hydrogen, C₁-C₈alkyl, allyl, cyclohexyl, benzyl or a group of the formula IIIa, in which R is hydrogen and R₈ has the meaning indicated in claim 5, and in particular is C₁-C₄alkyl or hydrogen.

7. A compound of the formula Ia according to claim 1, in which A is -CH₂CO-, R₁ and R₂ and also R₃ and R₄ in pairs are each hydrogen or C₁-C₄alkyl, R₅ and R₆ are each hydrogen, R₇ is a radical of the formula V, Q₁ is a direct bond, R₈ is hydrogen, C₁-C₄alkyl, benzyl, allyl, acetyl or acryloyl, and R' is hydrogen, methyl or phenyl.

8. A compound of the formula Ia according to claim 1, in which A is -CH₂CO-, R₁ and R₂ are each hydrogen and in particular each methyl, R₃ and R₄ are each methyl and R₅ and R₆ are each hydrogen and R7 is a radical of the formula V, in which Q₁ is a direct bond, R₈ is hydrogen, methyl, benzyl, allyl or acetyl, and R' is hydrogen or methyl.

9. A compound of the formula Ia according to claim 1, in which A is -CH₂CH(OH)CH₂-, R₁ and R₂ and also R₃ and R₄ in pairs are each hydrogen or C₁-C₄alkyl, R₅ and R₆ are each hydrogen, R₇ is a radical of the formula V, Q₁ is a direct bond, R₈ is hydrogen, C₁-C₄alkyl, benzyl, allyl, acetyl or -CH₂CH(OH)R'.

10. A compound of the formula Ia according to claim 1, in which A is -CH₂CH(OH)CH₂-, R₁ and R₂ are each hydrogen and in particular each methyl, R₃ and R₄ are each methyl and R₅ and R₆ are each hydrogen and R₇ is a radical of the formula V, Q₁ is a direct bond, R₈ is methyl, benzyl, allyl or -CH₂CH(OH)CH₃.

11. An organic material comprising at least one compound according to claim 1 as a stabiliser.

12. An organic material according to claim 11, in which the organic material is an organic polymer.

13. An organic material according to claim 11, in which the organic material is a paint.

14. An organic material according to claim 11, in which the organic material is a recording material.

15. The use of compounds according to claim 1 as stabilisers for organic materials against damage by light, oxygen and/or heat.

## Revendications

1. Composé de formule Ia 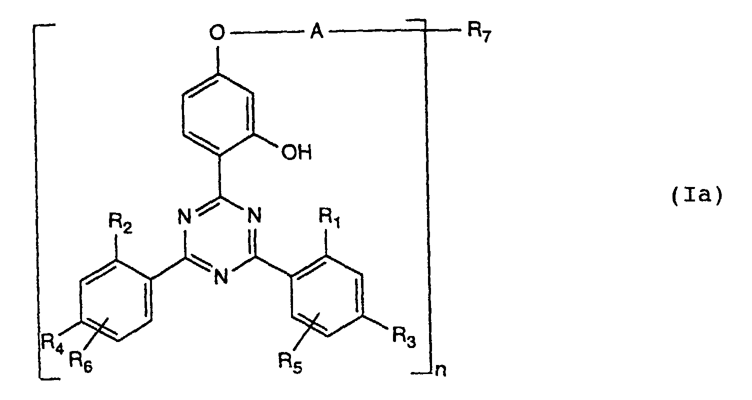 où
n vaut 1,
R₁ et R₂ représentent, indépendamment l'un de l'autre, des atomes d'hydrogène, des groupes -OH, alkyle en C₁-C₁₂ ou des atomes d'halogènes,
R₃ et R₄ représentent, indépendamment l'un de l'autre, des atomes d'hydrogène, des groupes -OH, alkyle en C₁-C₁₂, alkoxy en C₁-C₁₈, des atomes d'halogènes ou un reste -O-A-R₇,
R₅ et R₆ représentent, indépendamment l'un de l'autre, des atomes d'hydrogène, des groupes alkyle en C₁-C₁₂ des atomes d'halogènes ou un reste -O-A-R₇,
A représente une liaison directe, des groupes -(CH₂)ₘCO- avec m = 0 ou allant de 1 à 4, -CH₂CH(R')O- ou -CH₂CH(OH)CH₂-,
R₇ représente un reste de formules V à VIIb ou Xa ou Xb : 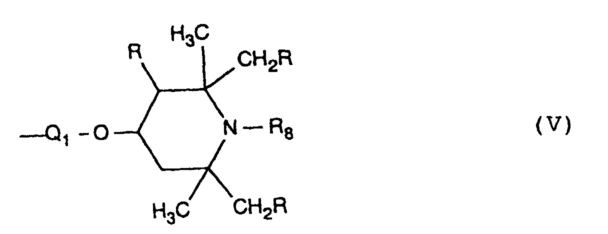 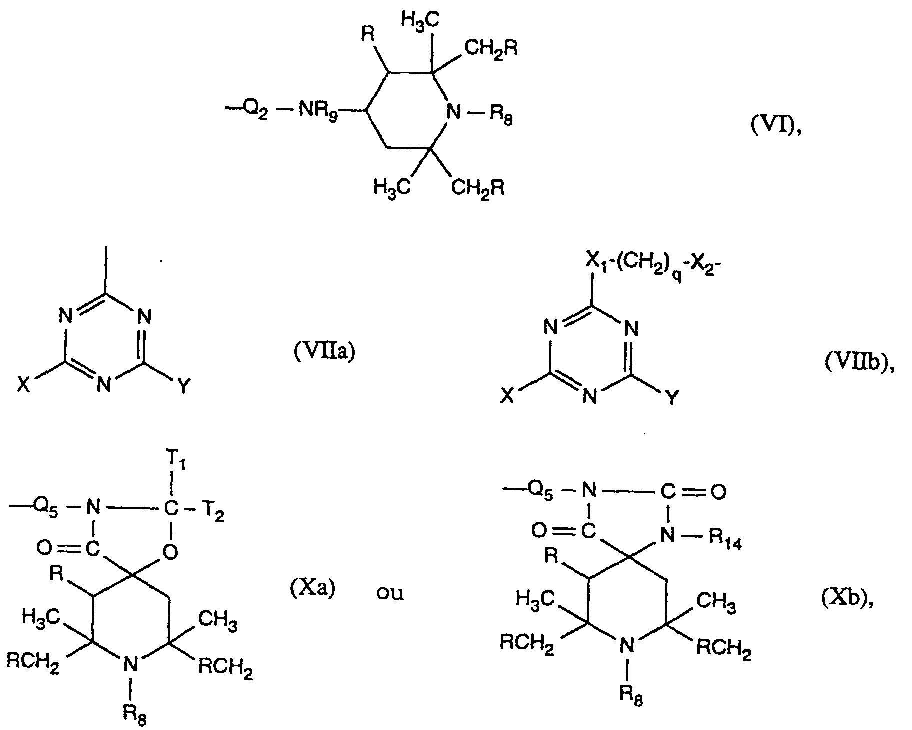 les restes de formules VIIa n'étant présents que dans des composés de formule Ia avec A = liaison directe ou -CH₂CH(R')-O- et des restes de formules VIIb n'étant présents que dans des composés de formule Ia, où A ne représente pas une liaison directe ou un groupe -CH₂CH(R')-O-, et où
R représente un groupe méthyle et en particulier un atome d'hydrogène,
R₈ représente un atome d'hydrogène, des groupes oxyle, alkyle en C₁-C₁₂, alkoxy en C₁-C₁₂, alcényle en C₃-C₈, alcényloxy en C₃-C₈, alcynyle en C₃-C₈, cycloalkyle en C₅-C₆, phénylalkyle en C₁-C₄, alcanoyle en C₁-C₈, alcénoyle en C₃-C₅, -CH₂CH(OH)R' ou phénoxy,
R' représente un atome d'hydrogène, un groupe méthyle ou phényle,
R₉ représente un atome d'hydrogène, des groupes alkyle en C₁-C₁₈, allyle, cycloalkyle en C₅-C₆, phénylalkyle en C₁-C₄, alkyle en C₃-C₈ interrompu par un ou plusieurs atomes -O- ou un groupe de formule IIIa 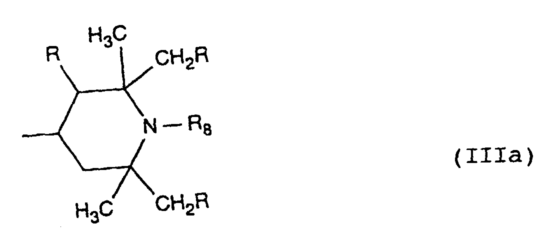
Q₁ représente la liaison directe, des groupes -O-alkylène en C₂-C₁₂ ou -NR₉-alkylène en C₂-C₁₂ lorsque A possède la signification donnée, mais A ne représente pas une liaison directe ou un groupe -CH₂CH(R')-O-,
ou dans les composés de formule Ia, Q₁ représente un groupe alkylène en C₂-C₁₂, lorsque A représente la liaison directe ou un groupe -CH₂CH(R')-O-,
ou représente un groupe -OCO-alkylène en C₂-C₁₂, lorsque A représente dans les composés de formule Ia un groupe -CH₂CH(OH)CH₂-,
Q₂ représente la liaison directe ou un groupe -NR₉-alkylène en C₂-C₁₂, lorsque A ne représente pas une liaison directe ou un groupe -CH₂CH(R')-O-, ou dans les composés de formule Ia, Q₂ représente un groupe -CO-alkylène en C₂-C₁₂, lorsque A représente la liaison directe ou un groupe -CH₂CH(R')-O-, ou représente un groupe -OCO-alkylène en C₁-C₁₂ ou -OCO-(alkylène en C₁-C₁₂)-CO-, lorsque A dans la formule Ia représente un groupe -CH₂CH(OH)CH₂-,
q va de 2 à 12, en particulier de 2 à 4,
X₁ et X₂ représentent, indépendamment l'un de l'autre, des groupes -O- ou R₉N-,
X 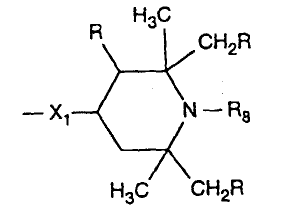 et Y possède la même signification que X ou représente un groupe alkoxy en C₁-C₁₈ ou -NR₉R₁₀,
R₁₀ peut posséder la même signification que R₉, ou forme, conjointement avec R₉ et l'atome d'azote de liaison, un cycle hétérocyclique à 5 ou 6 chaînons,
Q₅ représente un groupe -OCH₂CH₂- ou -OCH₂CH(OH)CH₂-, lorsque A ou B possèdent la signification donnée, mais A ne représente pas une liaison directe ou un groupe -CH₂CH(R')-O-,
ou dans les composés de formule Ia, Q₅ représente un groupe -CO(CH₂)₂- ou -CO(CH₂)₃-, lorsque A représente la liaison directe ou un groupe -CH₂CH(R')-O-,
Q₅ représente la liaison directe, lorsque A représente un groupe -CH₂CH(OH)CH₂-,
ou Q₅ représente un groupe -OCO(CH₂)ₛ- avec s = 1 à 4, lorsque A dans la formule Ia représente un groupe -CH₂CH(OH)CH₂-,
T₁ et T₂ représentent, indépendamment l'un de l'autre, des atomes d'hydrogène, des groupes alkyle en C₁-C₁₈, phénylalkyle en C₁-C₄ ou phényle ou naphtyle substitué par des atomes d'halogènes ou des groupes alkyle en C₁-C₄, ou T₁ et T₂ forment, conjointement avec l'atome de carbone qui les relie, un cycle cycloalcane en C₅-C₁₂,
R₁₄ représente un atome d'hydrogène, des groupes alkyle en C₁-C₁₂, allyle, benzyle ou alkoxyalkyle en C₂-C₆.

2. Composé selon la revendication 1, où R représente un atome d'hydrogène, R' représente un atome d'hydrogène ou un groupe méthyle et m et s valent chacun 1 et R₁ et R₂, R₃ et R₄ ainsi que R₅ et R₆, à chaque fois par paires, possèdent la même signification.

3. Composé selon la revendication 1, où R₁ et R₂, ainsi que R₃ et R₄, à chaque fois par paires, représentent des atomes d'hydrogène ou des groupes alkyle en C₁-C₄ et R₅ et R₆ représentent chacun un atome d'hydrogène.

4. Composé selon la revendication 1, où dans la formule Ia, R₁ et R₂ représentent chacun un atome d'hydrogène, un groupe -OH ou méthyle, en particulier un atome d'hydrogène ou un groupe méthyle, R₃ et R₄ représentent chacun un atome d'hydrogène, des groupes -OH ou méthyle, en particulier un atome d'hydrogène ou un groupe méthyle, et R₅ et R₆ représentent chacun un atome d'hydrogène.

5. Composé selon la revendication 1, où R₈ représente un atome d'hydrogène, des groupes oxyle, alkyle en C₁-C₁₂, alcényle en C₃-C₈, alcynyle en C₃-C₈, phénylalkyle en C₁-C₄, alcanoyle en C₁-C₈, alcénoyle en C₃-C₈ ou -CH₂CH(OH)R', en particulier un atome d'hydrogène, des groupes oxyle, alkyle en C₁-C₈, allyle, benzyle, acétyle ou acryloyle.

6. Composé selon la revendication 1 ou 5, où R₉ et R₁₀ représentent des atomes d'hydrogène, des groupes alkyle en C₁-C₈, allyle, cyclohexyle, benzyle ou un groupe de formule IIIa, où R représente un atome d'hydrogène et R₈ possède la signification donnée à la revendication 5, et représente en particulier un groupe alkyle en C₁-C₄ ou un atome d'hydrogène.

7. Composé de formule Ia selon la revendication 1, où A représente un groupe -CH₂CO-, R₁ et R₂, ainsi que R₃ et R₄ représentent, à chaque fois par paires, des atomes d'hydrogène ou des groupes alkyle en C₁-C₄, R₅ et R₆ représentent chacun un atome d'hydrogène, R₇ représente un reste de formule V, Q₁ est la liaison directe, R₈ représente un atome d'hydrogène, des groupes alkyle en C₁-C₄, benzyle, allyle, acétyle ou acryloyle, et R' représente un atome d'hydrogène, un groupe méthyle ou phényle.

8. Composé de formule Ia selon la revendication 1, où A représente un groupe -CH₂CO-, R₁ et R₂ représentent chacun un atome d'hydrogène et en particulier un groupe méthyle, R₃ et R₄ représentent chacun un groupe méthyle et R₅ et R₆ représentent chacun un atome d'hydrogène et R₇ représente un reste de formule V, où Q₁ est la liaison directe, R₈ représente un atome d'hydrogène, des groupes méthyle, benzyle, allyle ou acétyle et R' représente un atome d'hydrogène ou un groupe méthyle.

9. Composé de formule Ia selon la revendication 1, où A représente un groupe -CH₂-CH(OH)CH₂-, R₁ et R₂, ainsi que R₃ et R₄ représentent, à chaque fois par paires, des atome d'hydrogène ou des groupes alkyle en C₁-C₄, R₅ et R₆ représentent chacun un atome d'hydrogène, R₇ représente un reste de formule V, Q₁ est la liaison directe, R₈ représente un atome d'hydrogène, des groupes alkyle en C₁-C₄, benzyle, allyle ou acétyle, -CH₂-CH(OH)R'.

10. Composé de formule Ia selon la revendication 1, où A représente un groupe -CH₂-CH(OH)CH₂-, R₁ et R₂ représentent chacun un atome d'hydrogène et en particulier un groupe méthyle. R₃ et R₄ représentent chacun un groupe méthyle et R₅ et R₆ représentent chacun un atome d'hydrogène et R₇ représente un reste de formule V, Q₁ est la liaison directe, R₈ représente des groupes méthyle, benzyle, allyle ou -CH₂-CH(OH)CH₃.

11. Matière organique contenant comme stabilisant au moins un composé selon la revendication 1.

12. Matière organique selon la revendication 11, où la matière organique est un polymère organique.

13. Matière organique selon la revendication 11, où la matière organique est une peinture.

14. Matière organique selon la revendication 11, où la matière organique est une matière d'enregistrement.

15. Utilisation de composés selon la revendication 1 en tant que stabilisants de matières organiques contre la dégradation induite par la lumière, l'oxygène et/ou la chaleur.
